# EUROPÄISCHE PATENTSCHRIFT

(11) **EP 1 266 028 B1**
(45) Veröffentlichungstag und Bekanntmachung des Hinweises auf die Patenterteilung: **23.01.2008**
(21) Anmeldenummer: 00991559.6
(22) Anmeldetag: 27.12.2000
(51) Int. Cl.: C12Q 1/68, G06F 19/00

(54) **VERFAHREN ZUR SCHNELLEN BESTIMMUNG VON MIKROBIELLER DNS/RNS, KIT DAFÜR UND VERWENDUNG DES VERFAHRENS**
METHOD FOR QUICKLY DETECTING MICROBIAL DNA/RNA, KIT THEREFORE AND THE USE OF SAID METHOD
PROCEDE DE DETERMINATION RAPIDE DE DNS/RNS MICROBIENS, TROUSSE A CET EFFET ET UTILISATION DU PROCEDE

(30) Priorität: 23.12.1999 DE 19962895; 31.05.2000 DE 10027113
(43) Veröffentlichungstag der Anmeldung: 18.12.2002
(60) Teilanmeldung: 07001454.3
(73) Patentinhaber: Roche Diagnostics GmbH, 68305 Mannheim (DE)
(72) Erfinder: Hoeft, Andreas, Prof. Dr. med., 53127 Bonn (DE); Stüber, Frank, 53127 Bonn (DE)
(86) Internationale Anmeldenummer: PCT/DE2000/004610
(87) Internationale Veröffentlichungsnummer: WO 2001/048237

(56) Entgegenhaltungen:
- RIRIE ET AL.: "Product Differentiation by Analysis of DNA Melting Curves during the Polymerase Chain Reaction" ANALYTICAL BIOCHEMISTRY, 1997, Seiten 154-160, XP001015991
- NAUCK ET AL.: "Rapid, Homogenous Genotyping of the 4G/5G Polymorphism in the Promoter Region of the PAI 1 Gene by Fluorescence Resonance Energy Transfer and Probe Melting Curve" CLINICAL CHEMISTRY, Bd. 45, Nr. 8, 1. August 1999 (1999-08-01), Seiten 1141-1147, XP002175787
- AHSEN ET AL.: "Application of a Thermodynamic Nearest-Neighbor Model to Estimate Nucleic Acid Stability and Optimize Probe Design: Prediction of Melting Points of Multiple Mutations of Apolipoprotein B-3500 and Factor V with a Hybridization Probe Genotyping Assay on the LightCycler" CLINICAL CHEMISTRY, Bd. 45, Nr. 12, 1. Dezember 1999 (1999-12-01), Seiten 2094-2101, XP002175788
- DE SILVA: "Rapid Genotyping and Quantification on the LightCycler with Hybridization Probes" BIOCHEMICA, Nr. 2, 1998, Seiten 12-15, XP002175789
- SHEPERD ET AL.: "Monitoring of fluorescence during DNA melting as a method for discrimination and detection of PCR products in variety identification" MOLECULAR BREEDING, Bd. 4, Nr. 6, 1998, Seiten 509-517, XP001016033
- WOO ET AL.: "Identification of Leptospira biflexa by real-time homogenous detection of rapid cycle PCR product" JOURNAL OF MICROBIOLOGICAL METHODS, Bd. 35, Nr. 1, Februar 1999 (1999-02), Seiten 23-30, XP001016145
- KLAUSEGGER ET AL.: "Gram Type-Specific Broad Range PCR Amplification for Rapid Detection of 62 Pathogenic Bacteria" JOURNAL OF CLINICAL MICROBIOLOGY, Bd. 37, Nr. 2, Februar 1999 (1999-02), Seiten 464-466, XP002176097 in der Anmeldung erwähnt
- ANTHONY ET AL.: "Rapid Diagnosis of Bacteremia by Universal Amplification of 23S Ribosomal DNA Followed by Hybridization to an Oligonucleotide Array" JOURNAL OF CLINICAL MICROBIOLOGY, Bd. 38, Nr. 2, Februar 2000 (2000-02), Seiten 781-788, XP002176098 in der Anmeldung erwähnt
- BERNARD ET AL.: "Homogenous Multiplex Genotyping of Hemochromatosis Mutations with Fluorescent Hybridization Probes" AMERICAN JOURNAL OF PATHOLOGY, Bd. 153, Nr. 4, Oktober 1998 (1998-10), Seiten 1055-1061, XP000874040
- ESPY ET AL.: "Diagnosis of Herpes Simplex Virus Infections in the Clinical Laboratory by LightCycler PCR" JOURNAL OF CLINICAL MICROBIOLOGY, Bd. 38, Nr. 2, Februar 2000 (2000-02), Seiten 795-799, XP002175791
- PIETILÄ ET AL.: "Rapid Differentiation of Borrelia garinii from Borrelia afzelii and Borrelia burgdorferi Sensu Stricto by LightCycler Fluorescence Melting Curve Analysis of a PCR Product of the recA Gene" JOURNAL OF CLINICAL MICROBIOLOGY, Bd. 38, Nr. 7, Juli 2000 (2000-07), Seiten 2756-2759, XP002175790

## Beschreibung

Die Erfindung betrifft ein Verfahren zur schnellen Bestimmung von mikrobieller DNS/RNS; ein Kit dazu sowie die Verwendung des Verfahrens.

Die schnelle Bestimmung von Mikroorganismen, insbesondere der häufig auftretenden Bakterien, Viren und Pilzen, ist allgemein von Interesse, bspw. bei Lebensmitteln, auch Wasser, der Kriminalistik etc. Ein besonders wichtiges Einsatzfeld ist aber die klinische Diagnostik, insbesondere bei Intensivpatienten mit schweren bakteriellen Infektionen, die ein hohes Risiko aufweisen, im Rahmen der systemischen Entzündungsreaktion Organschäden davon zu tragen oder sogar daran zu versterben. Die Sterblichkeit der schweren Sepsis, des septischen Schocks und des Multiorganversagen beträgt bis zu 90%. Es stehen zur Zeit keine laborchemischen Frühparameter zur Verfügung, welche den Beginn einer Infektion, auch einer schweren systemischen Infektion anzeigen. Bisherige Routineentzündungsparameter wie Anstieg der Leukozytenzahl oder Anstieg des C-reaktiven Proteins reagieren erst mit einer zeitlichen Verzögerung von 1 bis 3 Tagen auf eine systemische Infektion, sind für diese nicht spezifisch und können auch ohne Infektion mit Mikroorganismen erhöht sein. Eine schnelle Bestimmung der Mikroorganismen ist aber auch bei lokalen Infektionen, bspw. Augeninfektionen - auch nach Augenoperationen - die sonst sogar bis zum Verlust des operierten Auges führen können - der Parodontitis, oder aber lokalen Pilzinfektionen äußerst wünschenswert. Eine frühzeitige Diagnose einer Infektion ist essentiell für die Möglichkeit einer frühzeitigen Therapie. Insbesondere ist es wichtig, den infizierenden Mikroorganismus zeitig zu identifizieren, um eine gerichtete Antibiotika-Therapie einleiten zu können. Die konventionelle mikrobiologische Diagnostik durch Anzucht und Kulturnachweis ist hierbei jedoch nicht befriedigend. Zum einen dauert dieser Nachweis häufig mehrere Tage, zum anderen ist diese Art des Nachweises nur möglich, wenn in der vorgelegten Probe vitale Bakterien enthalten sind. Da Körperflüssigkeiten, insbesondere Blut, bakterizide Eigenschaften aufweisen, ist dies jedoch häufig nicht der Fall auch wenn eine Infektion vorliegt. Die konventionelle mikrobiologische Diagnostik weist daher eine hohe falsch negative Befundrate auf Eine schnelle Diagnose einer Infektion sowie eine schnelle Identifizierung des infizierenden Mikroorganismus wurde daher die Möglichkeit einer frühzeitigen Therapie bedeuten, die speziell auf den aufgefundenen Organismen ausgerichtet ist und daher zu der Hoffnung berechtigt, die Krankheitsschwere oder sogar die Mortalitätsrate der Erkrankung günstig zu beeinflussen.

### Stand der Technik

Es ist bekannt, daß bakterielle oder auch Pilz-DNS/ mittels Konsensus-Primern unspezifisch durch PCR vermehrt werden können, so daß dann, wenn nur geringste Spuren der DNS/RNS vorhanden sind, aus diesen geringen Mengen für Nachweise ausreichende Mengen hergestellt werden (ROCHE-LightCycler-Anweisung). Die so in für übliche Nachweisverfahren in ausreichender Menge hergestellte doppelsträngige DNS kann dann als solche qualitativ, selektiv und quantitativ nachgewiesen werden.

Ein qualitativer Nachweis der Anwesenheit von mikrobieller DNS/RNS kann - bspw. durch Interkalations-Agentien, die nur an doppelsträngige DNS binden und bei der Bindung ein Signal, das Bindung anzeigt, abgeben, erfolgen. Ein spezifischer Nachweis kann auch über spezifische Sonden, die nur an die DNS/RNS bestimmter Spezies binden, erfolgen - falls ein anderer, als der angenommene, Mikroorganismus in der Probe anwesend ist, müssen so lange mit verschiedenen spezifischen Sonden Versuche durchgeführt werden, bis eine Sonde paßt, also eine spezifische Bindung anzeigt. Die bisherigen Verfahren liefern also im ersten PCR-Laufä nur die Information, daß RNS/DNS bakterieller oder pilz-Herkunft vorliegt, die Spezifikation selbst ist ein weiteres aufwendiges Suchverfahren. , das nur durch Erfahrungswerte bestimmt ist.

In der WO 97/07238 wurde die Vermehrung von Pilz-DNS aus klinischem Material - (Blut) mittels Konsensus-Primern, die einen Bereich der 18 ssu-rRNS mittels PCR amplifizieren und die spezifische Identifikation der vermehrten Pilz-DNS durch nachgeschalteten Southern Blot beschrieben. Auf die Ofenbarung dieser Druckschrift wird zur Erläuterung der PCR und der Vermeidung von Wiederholungen in vollem Umfang bezug genommen.

Bei Bakterien sind ebenfalls Konsensus-Primer bekannt, die an hochkonservierte Bereiche von Bakterien-DNS binden - bspw. die hochkonservierte 16 S Region der rRNS oder aber die ebenfalls hochkonservierte 23 S-Region der rDNS. Die entsprechenden Templates können mit geeigneten Konsensusprimern äamplifiziertä - werden und dann die so vermehrte Bakterien-DNS mittels verschiedener Nachweismethoden nachgewiesen werden (Anthony, Brown, French; J. Clin. Microbiol. 2000, S. 781 - 788 "Rapid Diagnosis of Bacteremia by universal amplification of 23S Ribosomal DNS followed by Hybridization to an Oligonucleotide Array und WO 00/66777) .

Die WO 00/66777 - wie auch bereits Woo, Patel et.al. in Anal. Biochem 1998, 259 sowie in J. Microbiol Methods 1999, 23 -30 - schlagen vor, bakterienspezifische Primer zu verwenden,; so nur spezifische Bakterien zu amplifizieren und dann den Schmelzpunkt der mit interkalierenden Substanzen - dort meist SYBR-GREENmarkierten einzigen durch die speziellen Primer verstärkten doppelsträngigen DNS zu bestimmen, um die mittels der spezifischen Primer amplifizierten DNS nochmals zu identifizieren, da die Schmelzpunkte von Hybridisationsprodukten Sonde/DNS typisch sind. Dies ist auch für Herpes-Viren - HSV-bekannt - Espyl, Uhl et.a. J. Clin. Microbiol. 2000, S. 795 f. - dort wird speziell für das Thymidin-Kinase Gen des Virus mit geeigneten Primern amplifiziert und nachfolgend mittels Schmelzkurvenanalyse die über die Primer identifizierte amplifizierte DNS nochmals überprüft. Die dort verwendeten spezifischen Primer binden nicht ausschließlich an Konsensus-Bereiche, sondern an ganz spezifische Sequenzen, sodaß nur diese amplifiziert werden. Mit diesem Verfahren kann die Anwesenheit weiterer Mikroorganismen nebeneinander nicht detektiert werden kann.

Klausegger et.al in J.Clin. Microbiol. 1999, S. 464-466 "Gram-Type Specific Broad-Range PCR Amplification for Rapid Detection of 62 Pathogenic Bacteria...zeigt, daß die DNS/RNS gram(+)er Bakterien mit speziell entworfenen gram-positiven Primern in einer PCR gruppen-spezifisch amplifiziert werden und die gram - Bakterien nicht - derart ist immerhin eine Unterteilung der zu untersuchenden Bakterien in gram+Bakterien und gram- Bakterien möglich.

Eine weitere Spezifizerung der so amplifizierten gram(+)erfolgt bei Klausegger, mit herkömmlichen mikrobiologischen Methoden. Nach Klaüsegger ist zumindest eine schnelle Behandlung pathogener Bakterien, die auf gram+ - oder gram-Bakterien gerichtet ist, möglich. Eine genauere Identifikation ist bei Klausegger aber nicht innerhalb kurzer Zeit' möglich.

Die Amplifikation bakterieller rRNS ist keineswegs auf den 16S oder aber 23S-Bereich beschränkt - es ist auch bekannt, daß bei Bakterien die "spacer-Region" zwischen den 16 S und 23S Genen in der prokaryonten rRNS mit speziellen, dafür geeigneten Primern zu amplifiziert werden kann (App..and Enviromental Microbiology, 1993, S. 945 - 952) - die "spacer-Region" liegt zwischen zwei hochkonservierten Bereichen, auf denen dann jeweils ein Primer liegt.

Die Verfahren nach dem Stand der Technik ermöglichten somit in einer einzigen PCR lediglich die Entdeckung von Mikroorganismen durch Konsensus-Primer ohne Klassifikation derselben oder aber nur die Detektion eines einzigen Mikroorganismus. Zur Bestimmung der Mikroorganismus-Spezies war immer noch ein aufwendiges Verfahren notwendig, da verschiedenste mögliche Sonden durchprobiert werden mußten oder aber zur Bestimmung der übliche mikrobiologische Nachweis geführt werden mußte.

Es ist nun Aufgabe der Erfindung die Quantifizierung und Qualifizierung mikrobieller DNS/RNS schneller als bislang zu ermöglichen.

Die Aufgabe wird erfindungsgemäß gelöst durch ein Verfahren mit den Merkmalen des Patentanspruches 1.

Zur Bestimmung mikrobieller DNS/RNS wird also eingesetzt:
- Vorlegen einer Probe mit RNS/DNS
- unspezifische Amplifikation der mikrobiellen DNS/RNS mittels eines PCR-Verfahrens (Polymerase Chain Reaction) mit generischen Konsensus-Primern
- Analyse der physikalischen Eigenschaften der amplifizierten KonsensusSequenz (z.B. durch Durchführung einer Schmelzpunktanalyse im SYBR-Green Format oder aber durch Binden von multifunktionellen Sonden)
- ggf Zugabe eines markierten Oligonucleotids/Sonde
- Weitere Spezifizierung durch Analyse der physikalischen Eigenschaften des DNS/RNS Oligonucleotidkomplexes, wie z.B. der Temperaturabhängigkeit der Hybridisierung oder aber durch Einbringen spezieller Sonden und Analyse des Bindungsverhaltens der Sonden an die DNS mittels Fltc, bspw. durch Schmelzpunktanalyse der Temperaturabhängigkeit des Sondenbindungsverhaltens zur weiteren Spezifizierung des zu identifizierenden Mikroorganismus

Es ist vorteilhaft, wenn das PCR-Verfahren ein Real-Time PCR-Verfahren ist, da dadurch gleichzeitig mit einer Quantifizierung der vorgelegten RNS/DNS in der Probe im selben PCR-Durchgang eine Spezifizierung des identifizierten Mikroorganismus durchgeführt werden kann. Insbesondere ermöglicht der Nachweis mikrobieller genomischer DNS mit der sehr sensitiven und quantitativen real time PCR eine sehr frühe laborchemische Detektion einer mikrobiellen Infektion.

Als zu untersuchende Proben eignen sich bspw. Körperflüssigkeiten, Abstriche oder auch homogenisiertes Gewebe, aber auch alle anderen Materialien, in denen Reste von DNS nachgewiesen werden sollen.. Da a priori in der Regel der infizierende Mikroorganismus nicht bekannt ist, ist es sinnvoll, bei der PCR Primer zu verwenden, mit denen möglichst unspezifisch eine Amplifikation von DNS/RNS bei möglichst allen für die Diagnostik in Betracht zu ziehenden Mikroorganismen möglich ist.

Ein weiterer Aspekt der Erfindung betrifft ein Kit zur schnellen Bestimmung von mikrobieller DNS/RNS in Proben mit- dekontaminierter Polymerase; Lösung mit einer Standard DNS/RNS-Konzentration einer bekannten Template-) DNS/RNS. Bei einer bevorzugten Ausgestaltung weist das Kit weiterhin mindestens eine DNS/RNS-Oligonucleotid-Sonde auf. Diese speziellen DNS/RNS-Sonden können auch einzeln für die Identifizierung bestimmter Mikroorganismen zum Einsatz im erfindungsgemääen Verfahren vertrieben werden.

Ferner betrifft die Erfindung auch ein Verfahren oder aber Programm nach Anspruch 12. Durch dieses Programm können Automaten, die zur Durchführung einer real time-PCR geeignet sind, ebenfalls zum Nachweis und zur Identifizierung von Mikroorganismen eingesetzt werden.

Die Besonderheit des erfindungsgemäßen Verfahrens liegt darin, daß zunächst bei unbekanntem Mikroorganismus eine unspezifische Amplifikation mit Konsensus Primern durchgeführt wird, wobei die DNA-Abschnitte, die zwischen dem Konsensus Primern amplifiziert werden, für die jeweiligen Bakterien spezifische Unterschiede aufweisen. Diese bakterientypischen Unterschiede können herangezogen werden, um (nach unspezifischer Amplifikation) eine Identifizierung und Spezifizierung des tatsächlich vorliegenden Mikroorganismus durchzuführen.

Nach unspezifischer Amplifikation der DNS/RNS der Probe kann eine Identifizierung und Spezifizierung des infizierenden Mikroorganismus mit verschiedenen Verfahren durchgeführt werden, von denen nachfolgend einige Beispiele aufgeführt sind:
A. Reverser Dot Blot
B. DNS/RNS Mikrochip bzw. Microarray-Technik
C. Analyse der Schmelzkurve der gesamten amplifizierten DNS/RNS-Sequenz
D. Hybridisierung des Amplifikates mit markierten Oligonucleotid-Sonden,vorteilhafterweise während einer PCR
E. Schmelzkurvenanalyse der hybridisierten Oligonucleotid-Sonden
F. Kombination der Verfahren unter C-E

### ad A

Die DNS/RNS Mikrochip-Technik ist bspw. als Cnvynne P. und Page G.Microarray analysis, the next revolution in Molecular biology Science 1999 bekannt oder auch bei Sinclair B. "Everything's Great When it sits on a Chip - a Bright future for DNS arrays", The Scientist 1999, May 24,13(11) 18-20 bekannt.

### ad B

Der reverse Dot Blot ist bekannt.

### ad C

Die einfachste und schnellste Methode eine grobe Vorklassifizierung des infizierenden Mikroorganismus durchzuf hren, besteht in der Analyse der Schmelztemperatur des gesamten Amplifikates. Hierdurch kann eine einfache Unterscheidung in Gruppen wie z.B. gram-positiver oder gram-negativer Bakterien durchgeführt werden.

### ad D

Des weiteren kann bei Durchf hrung einer real time-PCR eine Spezifizierung mit speziellen Fluoreszenzfarbstoff-markierten Oligonucleotiden durchgeführt werden. Weiterhin kann die kombinierte Information aus der Schmelzkurvenanalyse des amplifizierten DNS-Abschnittes sowie des Bindungsverhaltens an spezifische Oligonucleotid-Sonden zur genaueren Spezifizierung: herangezogen werden.

### ad E

Darüber hinaus kann die Information, die sich aus der Schmelztemperaturanalyse der Oligonucleotid-Sonden ergibt zusätzlich zur Spezifizierung herangezogen werden.

### ad F

Schlußendlich ist es möglich, die Information, die sich aus allen genannten analytischen Verfahren ergibt, in Kombination zu benutzen, um den infizierenden Mikroorganismus genauestens zu bestimmen.

Somit kann durch Auswahl spezieller Sonden und Analyse der Schmelzkurven der Hybridisierungsprodukte an die mikrobielle DNS sehr schnell ermittelt werden, welche DNS vorliegt. Dabei ist es nicht notwendig, für jedes Bakterium eine spezifische Sonde herzustellen sondern es können mittels einiger weniger Sonden und den Schmelzpunkten des Hybridisierungsproduktes Aussagen über die Mikroorganismusart getrofen werden. 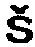 berraschenderweise ist es damit möglich, in nur einem Analysengang bei der Online-PCR nicht nur einen quantitativen Nachweis der mikrobiellen DNA durchzuf hren, sondern bereits im Anschluß an die Amplifikation durch Analyse mit wenigen Oligonucleotiden eine Identifizierung einer Vielzahl von verschiedenen Mikroorganismen herbeizuf hren.

Eine bevorzugte Verwendung des Verfahrens nach einem der Ansprüche ist die Schnellbestimmung von mikrobieller genomischer DNS insbesondere bei Intensivpatienten. Im Gegensatz zum konventionellen mikrobiologischen Nachweis mittels Kulturtechniken, die in der Regel mehrere Tage dauert, kann das hier dargestellte Verfahren mittels Online PCR in weniger als 3 Stunden durchgeführt werden. Hieraus ergibt sich eine enorme Beschleunigung der Diagnostik und somit eine wesentlich frühzeitigere Möglichkeit der gezielten Therapie.

Das Verfahren kann jedoch überall eingesetzt werden, wo schnell mikrobielle Kontamination quantifiziert und qualifiziert werden muß bspw. in der Kriminalistik aber auch in der Lebensmittelüberprüfung etc

Nachfolgend wird die Erfindung spezieller anhand der begleitenden Zeichnung sowie von Beispielen erläutert, auf die sie jedoch keineswegs eingeschränkt ist, die aber das Verständnis der Erfindung verbessern sollen. In der Zeichnung zeigt:
Fig. 1: Schmelzkurven von mit SYBR-Green markierten Gesamt-PCR-Produkten von 4 Bakterien, wobei die Fluoreszenz-Intensität als Funktion der Temperatur und die erste Ableitung dieser Kurve dl/dT dargestellt ist;
Fig. 1a: Darstellung der Schmelzpunkte verschiedener Bakterienarten mit SYBR-Green
Fig. 1b: Darstellung der Schmelzpunkte von Patientenproben mit SYBR-Green
Fig. 1c: Darstellung des PCR-Verlaufs der Patientenproben im SYBR-Green-Format
Fig. 2: Darstellung der Amplifizierung bakterieller DNS durch wachsendes Fluoreszenzsignal einer bindenden gram-neg. und gram-pos. Sonde am Ende der annealing-Phase des PCR-Zyklus;
Fig. 3: Schmelzkurven des Hybridisationsprodukts amplifizierter DNS mit einer spezifischen fluoreszenzmarkierten Hybridisierungssonde für gram-positive Keime (Fluoreszenz-Intensität in Abhängigkeit von der Temperatur bzw. die erste Ableitung der Kurve (darunter).
Fig. 4: Schmelzkurven wie in Fig. 3, aber mit einer Sonde für gram-negative Keime
Fig. 5: Schmelzkurven des Hybridisationsprodukts amplifizierter genomischer DNS verschiedener gram-negativer Keime mit gram-negativer Sonde -
Fig.6 Schmelzkurven d. Hybridisationsprodukts von mit markierten Primern amplifizierter genomischer DNS mit gram(+)- Sonden
Fig. 7 Schmelzkurven von Hybridisationsprodukten von. gram-pos Hyb-Probes mit Mischungen zwischen Pseudomonas und Staph.epi
Fig. 8 Schmelzkurven von Hybridisationsprodukten von gram-neg. Hyb-Probes mit Mischungen zwischen Pseudomonas und Staph.epi
Fig. 9. Schmelzkurven von BAL Patientenproben (P2 und P4) im Hyb-Probe-Format mit ISN2
Fig. 10: Schematische Darstellung des Amplifikationsverfahrens mit fluoreszeinmarkiertem Rückwärts Primer
Fig. 11. Schematische Darstellung der Lage erfindungsgemäáer Primer/Sondenpaare
Fig. 12: Alignment von bakterieller16rRNS mit Lage von Primern und Sonden
Fig. 13: Alignment von Pilz-RNS mit Lage von Primern und Sonden

### A Bestimmung des Mikroorganismus-Nucleotids

### Analyse der Schmelzpunkte doppelsträngiger Nucleotide aus Bakterien

Viele der nachfolgenden Versuche wurden mit Reinzucht-Bakterienstämmen aus der nachfolgenden Liste durchgeführt, die nach Erfahrungen der Erfinder in Kliniken sehr häufig aufzufinden sind, wobei die verwendeten Abkürzungen "RK" in den Zeichnungen, die Ausdrucke des Light-Cyder-Programms sind, auftreten:

| Abkürzg. | Mikroorganismus | ATCC-Nummer |
|---|---|---|
| RK1 | Pseudomonas aeruginosa | 27852 |
| RK2 | Klebsiella pneumoniae | 9591 |
| RK3 | Serratia marcescens | 264 |
| RK4 | Escherichia. coli | 25922 |
| RK5 | Proteus vulgaris | 6361 |
| RK6 | Haemophilus influenzae | 8142 |
| RK7 = | Enterococcus faecalis | 11420 |
| RK8 = | Enterococcus faecium | 19579 |
| RK9 = | Enterobacter aerogenes | 29007 |
| RK10 = | Enterobacter cloacae | 27508 |
| RK 11= | Streptococcus pyogenes | 8668 |
| RK12 = | Staphylococcus epidermidis | 14990 |
| RK13 = | Staphylococcus aureus. | 6538 |
| RK14 = | Bacteroides fragilis | 25285 |
| RK15 = | Mycobacterium tuberculosis | 25177 |
| RK16 = | Acinetobacter baumannii | 9955 |
| RK17 = | Legionella pnuemophila | 33152 |
| RK18 = | Candida albicans | 10231 |
| RK19 = | Aspergillus fumigatus | 1022 |

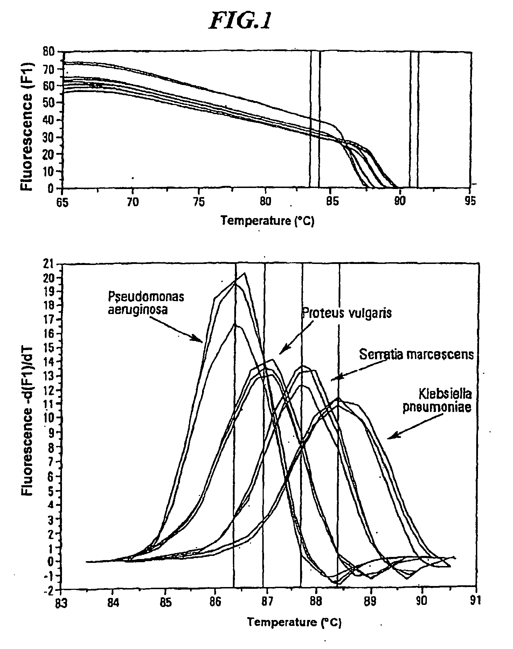

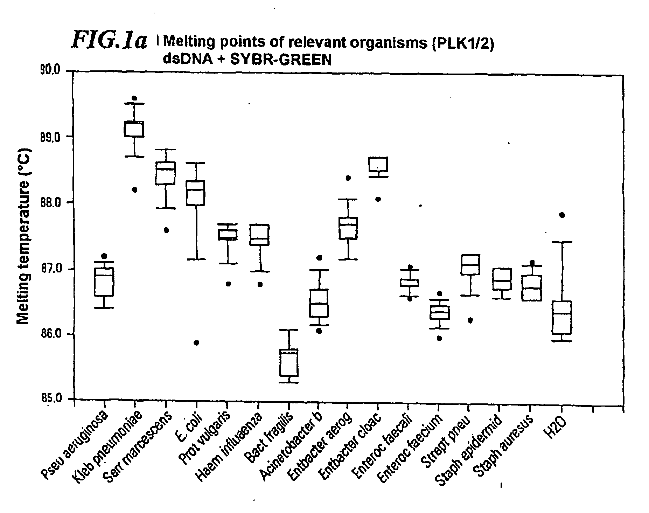

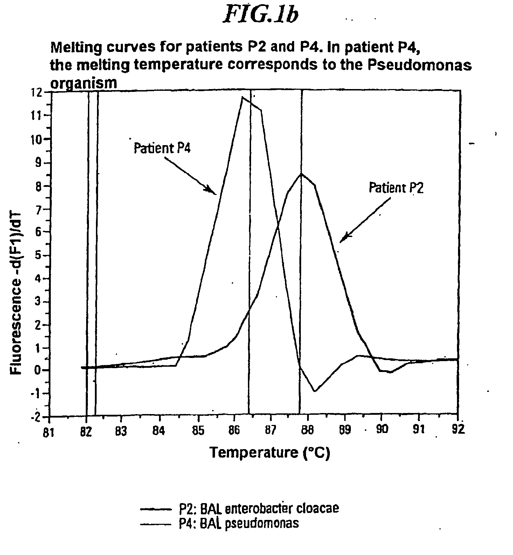

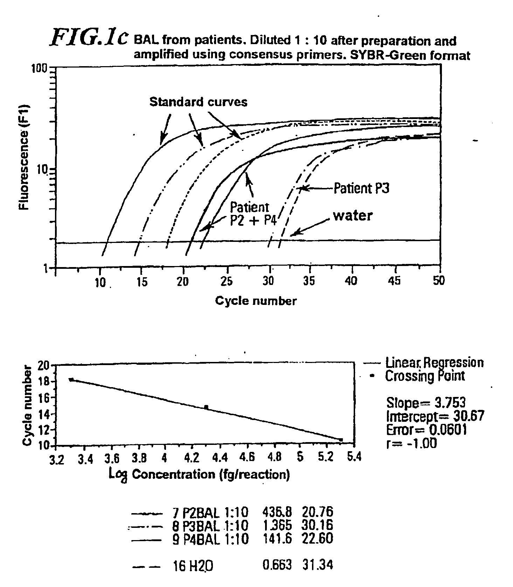

Fig. 1 zeigt die Schmelzkurvenanalyse des mit SYBR-Green markierten PCR-Produktes vier verschiedener Bakterien, wobei die Fluoreszenzintensität des SYBR-Green als Funktion der Temperatur bzw. die erste Ableitung der Intensität nach der Temperatur (im unteren Teil der Abbildung) dargestellt ist. Es wurde jeweils DNS eines Reinzucht-Stammes der nachfolgenden Bakterien wie in Bsp. Beschrieben über YY Zyklen amplifiziert und am Ende der Amplifikation eine Schmelzkurve mit SYBR-Green ermittelt. Die Mitte des Peaks wurde als Schmelzpunkt angenommen. Aus Fig. 1 wird deutlich, daß auch bei Verwendung eines Konsensus Primers - in diesem speziellen Fall für den 16-S-RNS-Bereich - die resultierenden PCR-Produkte - doppelsträngige DNS - für unterschiedliche Bakteriengruppen unterschiedliche physikalische Eigenschaften, insbesondere unterschiedliche Schmelztemperaturen aufweisen. Dies ergibt sich auch aus Fig. 1a, in der die Schmelzpunkte doppelsträngiger DNS von fünfzehn der üblichsten im Klinikbereich auftretenden Bakterien aufgeführt sind. Während für Pseudomonas aeruginosa die Schmelztemperatur im Bereich von 86,35 Grad C liegt, lassen sich hiervon deutlich die Schmelztemperaturen von Proteus vulgaris (ca 86,9 Grad C), Serratia marcescens (ca 87,75 Grad C) und Klebsiella pneumonia (ca 88,4 Grad C) diskriminieren. Es ist somit bereits an Hand der Schmelztemperaturkurvenanalyse des PCR-Amplifikates möglich, eine Gruppeneinteilung der möglicherweise vorhandenen Bakterien in mindestens 4 Gruppen vorzunehmen. Die Reproduzierbarkeit der Schmelzkurven ist ausgezeichnet, wie sich aus der nachfolgenden Tabelle ergibt.

### Reproduzierbarkeit der Schmelzpunkte:

| Bakterium | Versuch Nr. | Smpkt der dsDNS |
|---|---|---|
| Pseudomonas aer. | 1 | 86.36 |
| Pseudomonas aer. | 2 | 86.36 |
| Pseudomonas aer. | 3 | 86.34 |
| Klebsiella pneum. | 1 | 88.49 |
| Klebsiella pneum. | 2 | 88.42 |
| Klebsiella pneum. | 3 | 88.36 |
| Serratia marc. | 1 | 87.78 |
| Serratia marc. | 2 | 87.70 |
| Serratia marc. | 3 | 87,71 |
| Proteus vulg. | 1 | 86.92 |
| Proteus vulg. | 2 | 86,90 |
| Proteus vulg. | 3 | 86.93 |

### Design von Sonden/Primerkombinationen

Da erfindungsgemäß Mikroorganismen-DNS/RNS unspezifisch amplifiziert wird, ist die Auswahl bzw. der Entwurf geeigneter Primer und Sonden wichtig für die genauere Einordnung der Mikroorganismen und damit der Durchführung der Erfindung. Für Vorschläge zur Auswahl bzw. das Design von Primern und Sonden sind Programme, bspw. das Shareware Programm "GENE-FISHER" hilfreich, das bspw. von................. erhältlich ist. Dieses Programm sucht Konsensus-Bereiche von DNS / RNS und schlägt dann entsprechende Primer nach Vorgabe vor oder aber Sonden nach Vorgabe des Bereichs und deren Eigenschaften.

### Primer

Es ist bekannt, daß bestimmte Gensequenzfragmente innerhalb der meisten Bakterien bzw. den meisten Pilzen im wstl. gleich sind - diese werden als konservierte Bereiche bezeichnet. Aufgrund dieser konservierten Bereiche können mit "Konsensus-Primern" , die an diese Bereiche binden, die RNS/DNS dieser Mikroorganismen unspezifisch amplifiziert werden, wobei hier bei einer bevorzugten Ausführungsform der Erfindung zwischen den konservierten Bereichen ein für den Mikroorganismen spezifischer Bereich liegen soll, an den dann spezialisierte Sonden binden können. Derartige Bereiche sind bekannt und der Fachmann kann dann geeignete Bereiche für Primer aussuchen. Es ist wichtig, daß die Primer nicht zu lang sind, u. a. da dann die erwüschte Unspezifität nicht mehr gegeben ist.

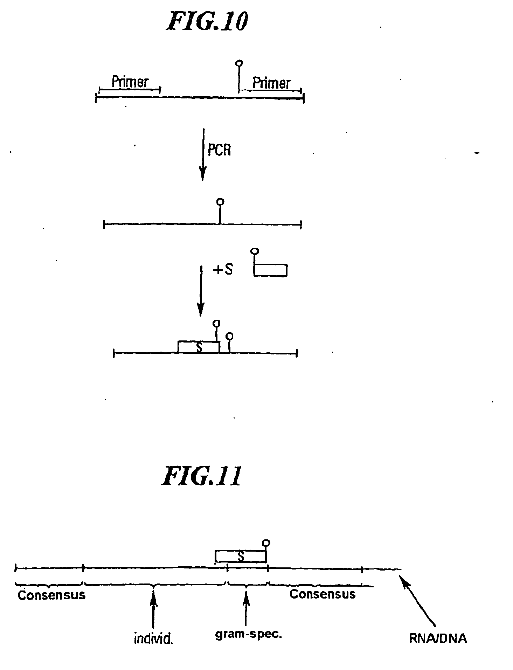

Erfindungsgemäß werden Konsensus-Primer verwendet, die einen hochvarianten und hochspezifischen Bereich flankieren, der für spezifischen Nachweis des Mikroorganismus geeignet ist (Fig. 11 und Fig. 12) Aus Fig. 12, die ein Alignment der entsprechenden RNA-Region verschiedener Bakterien darstellt, ist ersichtlich, wie die Primer sowie die gram(+) Sonde XXXX und die gram(-) Sonde liegen. Der Abstand zwischen beiden Primern ist bevorzugt nicht zu groß - zwischen etwa 100 und 250 Basenpaare auf dem zu amplifizierenden Template, um auch unvollständige fragmentierte DNS/RNS-Stücke, die in der zu untersuchenden Probe vorliegen, amplifizieren zu können. Auch die Primer selbst sollen nicht zu lang sein, da dies zu interner Loop-Bildung und anderen sterischen Veränderungen Störungen führen kann, welche die Hybridisierungseigenschaften nachteilig beeinflussen. Hier wurden Primer mit etwa 16 bis etwa 25 bp als sinnvoll angesehen

Es ist überraschenderweise auch gefunden worden, daß in der Praxis häufig nur Bruchstücke von RNS/DNS vorliegen, die durch große Primer nicht amplifiziert werden können, aber der Amplifikation von kurzen Primern zugänglich sind. Dennoch ist deren Auffinden wichtig, da es zu einer schnelleren und effektiven Behandlungsmöglichkeit führen kann. Gemäß einem Aspekt der Erfindung sind die Primer gelabelt - bspw. ist der Rückwärts Primer im Bereich der letzten Base mit einem Fluoreszenz-transferfähigen Farbstoff, wie Fluoreszein, gelabelt - dadurch enthalten alle amplifizierten Nucleinsäuren das Label am Konsensus-Bereich dieses Primers - wobei hier bevorzugt ein fluoreszierendes Label eingesetzt wird, das sich für FRET eignet, wie Fluoreszein, das an Thymidin gebunden sein kann.

Ein Konsensus-Bereich der hier beispielhaft genannten 16S-Region ist bspw. 16 - 20 Basenpaare lang - und verläuft bei E.coli um das bp 350 (s. Fig. 12), während der untere Konsensus-Bereich um das Basenpaar 525 liegt - an den unteren Bereich schließt sich - in 5'-Richtung gesehen - ein Unterscheidungs-Bereich an, der innerhalb der gram(+) Bakterien und der gram(-)Bakterien starke Ähnlichkeiten aufweist und etwa 3 Basenpaare lang ist.

Wegen der internen Loop-bildung sollten diese Konsensus-Primer für die 16S-Region zwischen etwa 16 bis etwa 25 bp lang sein. Die zwischen den hochkonservierten Bereichen der Primerbindungsstellen liegende variable bakterienspezifische Region ist ca 132 bp lang und hochspezifisch in ihrer Basensequenz für das Genom verschiedener Bakterienspezies.

Bei einem bevorzugten Ausführungsbeispiel wurden als Vorwärts-Primer (PrimerLänge: 16-18 Basen) für bakterielle 16rRNS solche gestaltet, die an den 16rRNS-Bereich des gram-Bakteriums E. coli im Bereich der konservierten Region bp 341 - 368 binden, während der dazugehörige Rückwärts-Primer im Bereich der konservierten Region bp 518-535 (Primerlänge: 16-28 Basen) bindet. Dies bedeutet, daß das Amplikon der PCR bis etwa 200 bp, bevorzugt kürzer ist.

Erfindungsgemäß ausgewählte Konsensus-Primer für die 16S-Region sind die im Sequenzprotokoll angegebenen Paare:
PLK1 als Vorwärts-Primer, der an die Basenpaare 341 - 368 der E.coli bindet, mit 16- 17 bp und PLK2 als Rückwärts Primer, der an die Bäsenpaare 518 - 535 der E.coli bindet, mit 17 bp., oder der Rev PLK2 mit 18 bp (1 Guanidin länger als PLK2) Als Vorwärts Primer eignen sich auch FOR mit 19 bp oder PRIMER A:mit17 bp

Der Fachmann kann auf Grundlage der obigen Anleitung jederzeit weitere geeignete Primer für hochkonservierte Regionen von mikrobieller DNS/RNS zu finden, die multivariante Bereiche, die für die Bestimmung der Spezies dienen können, umfassen. Obige Primer sind nur bevorzugte Ausführungsbeispiele für Bakterien, falls die 16s rRNA amplifiziert werden soll - falls die 23s rRNS Region zu amplifizieren ist, können nach dieser Anleitung andere Primer ausgewählt werden - dies gilt auch für andere hochkonservierte Bereiche von Bakterien rRNS, wie 5s RNS. Schließlich kann auch die "Spacer-Region" zwischen diesen Bereichen amplifiziert werden - bspw. der Bereich der Position 1493 -1513 der 16s rRNA von E.coli für einen A1-Primer und der Bereiche der Position23 - 43 der 23s rRNS von E.coli.für den dazugehörigen B1-Primer - wie von Barry e. al. In PCR Methods and Applications " Cold Spring Harbor Laboratory Press, ISSSN 1054-9803/91, S. 51 - 56 angegeben. Für Pilze eignen sich bspw. solche, die auf die Basenpaare 544 - 563 von C.Albicans bzw. bp 1033 - 1014 passen und 18ssu-rRNS - Gen -Sequenz-Teile amplifizieren. Ein weiterer Bereich betrifft die Amplifizierung und Analyse des Topoisomerase II Gens, welches ebenfalls zur Spezifizierung von Bakterienarten genutzt werden kann (Ragimbeau et al., J Appl Microbiol 1998;85(5):829-838).

### Sonden

Die Sonden können in an sich bekannter Weise für die Blot-Technik ausgelegt, gelabelt oder ungelabelt sein. Es können zwei zusammengehörige Sonden zum Nachweis einer Gruppe oder Art eingesetzt werden - bei einer speziellen Variante der Erfindung sind sie so ausgelegt, daß sie benachbart eines Konsensus-Bereiches binden und an dem dem Konsensus-Bereich benachbarten Ende für FRET (Fluorescence -Transfer - Coupling) mit einem entsprechendem Farbstoff gelabelt sind.

Unter hochspezifischen Sonden werden im Zusammenhang dieser Anmeldung solche verstanden, die exakt einer bestimmten Nucleotidsequenz eines Mikroorganismus entsprechen und nur an dieses Template einer einzigen Spezies binden.

Unter gruppenspezifische Sonden werden im Rahmen dieser Anmeldung solche verstanden, die an eine Gruppe von Spezies mit gemeinsamen Merkmalen binden - bspw. gram(+)-Sonden, die an gram(+)Bakterien bindet, da der Bindungsbereich der gram(+) Sonde zu untersuchende Bereich für gram+ Bakterien übereinstimmende Nucleotidsequenzen aufweist. Diese Sonden sind an den Bereich angepaßt und unterscheiden sich in einigen wenigen Mismatches von einer vollständigen Anpassung. Das gleiche gilt für das Pilzsondenpaar HPA und HPS, die untereinander FRET-fähig sind, also dann, wenn sie aneinander benachbart binden, den FRET-Effekt durch Fluoreszenz bei 640 nm zeigen. HPS und HPA sind typische Vertreter des 2-Sondensystems, das im erfindungsgemäßen Verfahren ebenfalls einsetzbar ist.

### Bakterien-Sonden

### Gruppen-Sonden

Wie sich aus Fig.10 und 11 ergibt, schließt an mindestens einen der beiden o.g. Konsensus-Bereiche der Bakterien 16rRNS beim bp der E.coli ein Unterscheidungsbereich gram(+)/gram(-) an, der hier ca 3 Basenpaare lang ist (gramspezifischer Bereich). Erfindungsgemäß werden verschiedene, Sondentypen eingesetzt.

Sonden für die FRET-Technik sind solche, die an einem Ende mit einem Fluoreszenz-Transferfähigen Farbstoff markiert sind, bspw. RBB 740 oder RBB 640 (zum Bsp.."Light Cycler System - LightCycler Instrument- Beschreibung - Abschnitt 6 - erhältlich von Roche Diagnostics GmbH, Sandhofer Str. 116, D-68305 Mannheim, Deutschland) . Falls der farbstoffgelabelte Bereich der Sonde in die Nähe eines fluoreszenzübertragenden anregbaren Moleküls, wie bspw. Fluoreszein kommt, findet bei Anregung des Fluoreszeins Fluoreszenztransfer des angeregten Fluoreszeins auf einen dadurch anregbaren Farbstoff, bspw. das RBB 740, RBB640 oder einen anderen Farbstoff statt, der dann emittiert. Die Voraussetzung für das Fluoreszenzsignal ist also die räumliche Nähe zwischen Fluoreszein und dem Transfer-Farbstoff, sonst tritt der Effekt nicht auf. Fluoreszein kann dabei an eine zweite Sonde (2-Sonden-Technik) gebunden sein, wobei dann beide Sonden an benachbarte spezifische Regionen binden müssen, damit der Effekt (Fluoreszenz in einem bestimmten Wellenlängenbereich) auftritt.

Unter hochspezifischen Sonden werden im Zusammenhang dieser Anmeldung solche verstanden, die exakt einer bestimmten Nucleotidsequenz eines Mikroorganismus entsprechen und nur an dieses Template einer einzigen Spezies binden.

Unter gruppenspezifische Sonden werden im Rahmen dieser Anmeldung solche verstanden, die an eine Gruppe von Spezies mit gemeinsamen Merkmalen binden - bspw. gram(+)-Sonden, die an gram(+)Bakterien bindet, da der Bindungsbereich der gram(+) Sonde zu untersuchende Bereich für gram+ Bakterien übereinstimmende Nucleotidsequenzen aufweist. Diese Sonden sind auf den Bereich angepaßt und unterscheiden sich in einigen wenigen Mismatches von einer vollständigen Anpassung.

Erfindungsgemäß wurden nun neue Sonden entwickelt, die auch einzeln einen Nachweis über FRET ermöglichen. Diese werden nachfolgend als "Single-Sonden" bezeichnet.

Dabei ist ein anregbarer Farbstoff am Rückwärts-Primer an dessen letzte Base gebunden, sodaß bspw. ein Fluoreszein-Label am Ende des Konsensusprimers bei der Amplifikation auf der diesem entsprechenden Konsensussequenz der neuen RNS am Ende des hochsensitiven Bereichs zu liegen kommt. Die passende Sonde kann nun so ausgelegt sein, daß sie direkt neben dem durch den Rückwärts-Primer eingegrenzten hochspezifischen Bereich an die Sequenz bindet und an ihrem zum Rückwärts-Primer gerichteten Ende mit einem FRET-anregbaren Farbstoff gelabelt ist. Falls die FRET-fähige Single-Sonde also in der Nähe der mittels des markierten Konsensusprimers amplifizierten Sequenz bindet, ist Fluoreszenztransfer möglich und die DNS nachweisbar. Zum Nachweis des Bindens einer Single-Sonde ist es - gegenüber dem Stand der Technik, der stets zwei Sonden im hochvariablen Bereich zum Nachweis benötigte, bei Verwendung der erfindungsgemäßen Kombination von mit fluoreszenzfähigem Farbstoff markierten Rückwärts-Primern und Single-Fluoreszenzsonden nur noch notwendig, eine einzige markierte Single-Sonde einzusetzen - der Aufwand an Sonden kann erheblich erniedrigt werden.

Selbstverständlich können auch anders gelabelte Sonden, bspw. für die Blot-Technik oder aber auch Sonden für die 2-Sonden-Technik eingesetzt werden, falls eine andere Sonden-Technik durchgeführt werden soll (wird im Zusammenhang mit Pilz-DNS-Nachweis nachstehend beschrieben).

### Sonden für gram+ und gram- Bakterien

Es wurde überraschenderweise gefunden, daß auch in dem hochvariablen Bereich zwischen den beiden Primersequenzen der 16srRNS kürzere Sequenzbereiche auftreten, die die gram+ und gram- Bakterien gemeinsam haben. Sonden für eine dieser beiden Gruppen müssen also die für die nachzuweisende Mikroorganismen-Gruppe gemeinsame spezifische analoge Sequenz besitzen. Falls bspw.bei gram-Bakterien die Identifikation. über den 16RNS-Bereich durchgeführt werden soll, eignen sich bspw. die nachfolgenden erfindungsgemäßen Sonden:
Typische Single-Sonden für die 16 S RNS von Gram-positiven Bakterien sind:ISP2 mit 30 Basen oder ISP mit 24 Basen
Typische Single-Sonden für Gram-negative Sonden ISN2 mit 28 Basen oder ISN mit 23 Basen

Bspw. binden die gruppenspezifischen Single-Sonden ISN2 für gram- Bakterien mit der Sequenz in 5'-3' Richtung von accgcagaataagcäccggctaacgtgc X,wobei X = LC640 an den Bereich der bp 467 - 497 des 16rRNS-Gens bei E. coli, die für im wesentlichen alle gram - Bakterien auf dem 16sRNS Gen gleich ist::
Single-Sonden für gram+ Bakterien binden an die bp von E. coli

Die Gruppenspezifische Single-Sonden zum Auffinden von gram+ Bakterien ISP2* bindet an den Bereich der bp 467 - 497 des 16rRNS-Gens bei E. coli, die für im wesentlichen alle gram - Bakterien auf dem 16sRNS Gen gleich ist::

Beide Sonden müssen mit einem Rückwärts-Primer wie PLK2* verwendet werden, der bei bp 517 der E. coli endet und in dieser Region mit Fluorescein gelabelt ist

Die hier verwendeten gruppenspezifischen Sonden sind über die gemeinsame Sequenz der Gruppe hinaus verlängert worden oder weisen einige wenige Mismatches - bevorzugt 1-4 und besonders bevorzugt 1 - 3 bei einer Sondenlänge von ca 25-30 bp auf, so daß bei verschiedenen Bakterien der Mikroorganismen-Gruppe unterschiedlich viele Mismatches auftreten. Diese Mismatches lassen sich dann u.a.. durch unterschiedliche Schmelzpunkte und Schmelzkurven des Hybrids mit dem DNS-Amplifikat nachweisen und erlauben eine Differenzierung der anwesenden Bakterien-RNS.

Weitere Beispiele für keimspezifische Sonden und Konsensus-Primer können - wobei die Erfindung keinesfalls auf diese beschränkt ist, sind die nachfolgend aufgeführten Oligonucleotide:

### Liste 1

Basensequenzen von Beispielen von als Sonden und Primer anwendbaren Oligonucleotiden in 5' nach 3' Richtung:

| Nucleot. Keim spezifische Sonden Bakterien | Sequenz (5'-3'-Richtung) |
|---|---|
| SLK 1 E. coli | agggagtaaagttaatacctttgctc |
| SLK 2 Staph. epi | gaacaaatgtgtaagtaactatgcacg |
| SLK 3 Pseudomonas | ggaagggcagtaagttaataccttg |
| SLK 4 Acinobacter baumanii | atacctagagatagtggacgttactc |
| SLK 5 Staph. aureus | gaacatatgtgfaagtaactgtgcaca |
| SLK 6 Enterococcus faecium | gatgagagtaactgttcatcccttg |
| SLK 7 Enterococcus faecialis | gacgttagtaactgaacgtcccct |
| SLK 8 Haemophilus influ. | tgatgtgttaatagcacatcaaattgac |
| SLK 9 Enterobacter cloacae | gacagggttaataaccctgtcgatt |
| SLK 10 Klebsiella pneumoniae | cgatgaggttaataacctcatcgatt |
| SLK 11 Serratia | aatacgctcatcaattgacgttactc |
| SLK 12 Legionella pneu. | ggttgataggttaagagctgattaac |
| SLK 13 Proteus vulgaris | tgataaagttaatacctttgtcaattgac |
| SLK 14 Bacteroides fragiles | tgcagtatgtatactgttttgtatgtatt |
| SLK 15 Streptococcus pyogenes | gtgggagtggaaaatccaccaagt |
| SLK 16 Mycobact. pneum. | gtaatggctagagtttgactgtacca |
| SLK 17 Corynebact. Jejuni | cactgtgtggtgacggtacctg |
| SLK 18 Enterobacter aerog | accttggcgattgacgttactcgc |
| SLK 19 Mycobact. tuberculosis gruppenspez. Sonden Bakterien (gram+/gram-) | ctctcggattgacggtaggtggag |
| SKNI gram(-)Sonde | gaggcagcagtggggaatattg |
| ISN2 gram(-)Sonde | accgcagaataagcaccggctaactccgtgcX, |
| | wobei X = LC640 |
| ISN2* gram(-)Sonde | ccgcagaataagcaccggctaactccgtX |
| | wobei X = LC640 |
| ISN gram(-)Sonde | agaagcaccggctaactccgXtgc |
| SKPI gram-positiv-Sonde | gaggcagcagtagggaatcttc |
| ISP2* gram-positiv-Sonde, gelabelt | cctaaccagaaagccacggctaactacgtg X, |
| | wobei X= LC705 |
| ISP2 gram-positiv-Sonde, gelabelt | cctaatcagaaagcgacggctaactacgtgcX |
| | wobei X= LC705 |
| ISP gram-positiv Sonde, gelabelt | agaaagccacggctaactacgXtgc |
| | wobei X= LC705 |

| Vorwärts-Primer Bakterien 16rRNS: | |
|---|---|
| PLK1H | tacgggaggcagcagt |
| FOR | tcctacgggAggcagcagt |
| PLK1 | ctacgggaggcagcagt |
| AR3: | gcg gtg aaa tgc gta gag at |

| Rückwärts-Primer Bakterien 16 rRNS: | |
|---|---|
| PLK2H | tattaccgcggctgctX |
| REV iFL, gelabelt | gtattaccgcggctgcXtg |
| Primer iFL | tattaccgcggctgcXtg, wobei X = Fluoreszein |
| AR4: | gtt tac ggc gtggactacca |

| Vorwärts-Primer Pilze: | |
|---|---|
| PFUI Pilz-Konsensus-Primer | attggagggcaagtctggtg |
| Rückwärts-Primer Pilze: | |
| PFU2 Pilz-Konsensus-Primer | ccgatccctagtcggcatag |

| Sonden für Pilze: | |
|---|---|
| spezifische Sonden: | |
| Scal Candida albicans | tctgggtagccatttatggcgaaccaggac |
| SCKI Candida krusei | gtctttccttctggctagcctcgggcgaac |
| SCPI Candida parapsilosis | tttccttctggctagcctttttggcgaacc |
| Sctl Candida tropiealis | gttggccggtccatctttctgatgcgtact |
| Stg1 Torulopsis glabrata | ttctggctaaccccaagtccttgtggcttg |
| SAUI Aspergillus | catggccttcactggctgtggggggaacca |

| gruppenspez. Sonden: | |
|---|---|
| HPA allgem.Pilzsondel | ctgaatgattaatagggacggtcgg-Fluorescein |
| HPS allgem.Pilzsonde 2 | LC640-ggtatcagtattcagttgtcagaggtgaaa |

Die Erfindung kann mit den oben aufgeführten Oligonucleotiden verwirklicht werden, sie ist aber keineswegs auf die oben beispielhaft aufgeführten Mikroorganismen und Nucleotide eingeschränkt, sondern läßt sich, mutatis mutandis, wie dem Fachmann offensichtlich ist, auch auf weitere Mikroorganismen mit anderen Nucleotide anwenden.

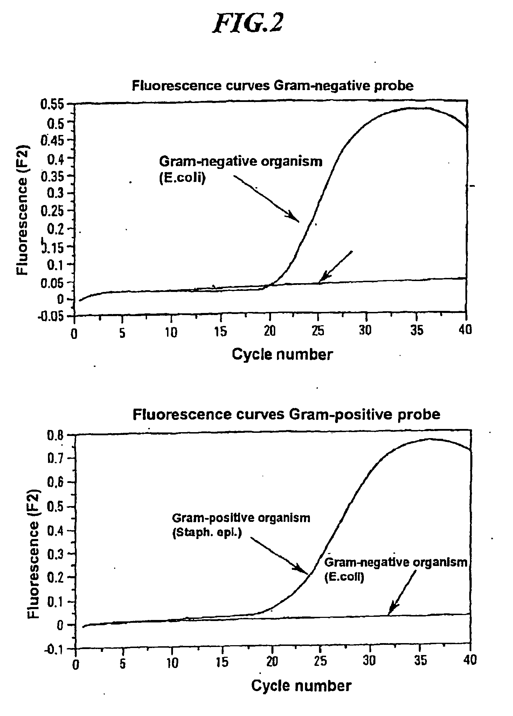

Fig.2, 3 und 4 stellen Möglichkeiten dar, die sich durch Verwendung von Oligonucleotid-Sonden bei der PCR ergeben. Hier handelt es sich um spezifische neue Sonden: ISN1 für gram-negative Bakterien und die gruppenspezifische Sonde ISP für gram-positive Bakterien. Dargestellt sind als Beispiel DNS von E. coli und Staph.epidermidis. Während die gram-negative Sonde bei der PCR nur ein Signal von E. coli liefert, ergibt sich mit dieser Sonde bei der Probe mit DNS von Staph.epi. kein Signal (Fig.2 oberer Teil). Umgekehrt identifiziert die Oligonuleotid-Sonde für gram-positive Bakterien eindeutig das gram+-Staph. epi, ist aber für die Probe mit DNS von E. coli negativ

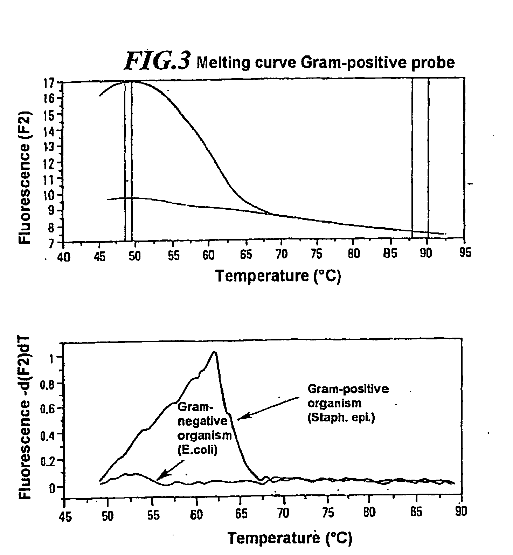

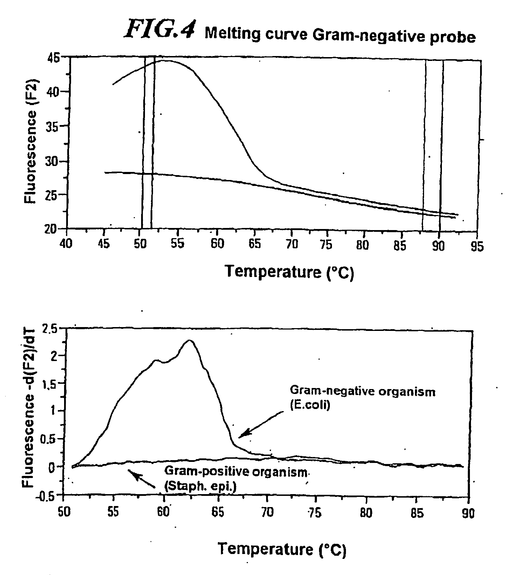

Fig.3 und Fig. 4 stellen für die gleichen Sonden die Schmelzkurvenanalyse dar. Auch an Hand der Schmelzkurven kann eine eindeutige Unterscheidung mit Hilfe der Oligonucleotide durchgeführt werden. Darüber hinaus ist es möglich, auch innerhalb der gram-negativen Bakterien an Hand der Schmelzkurvenanalyse der Sonde für gram-negative Bakterien weiter zu differenzieren.

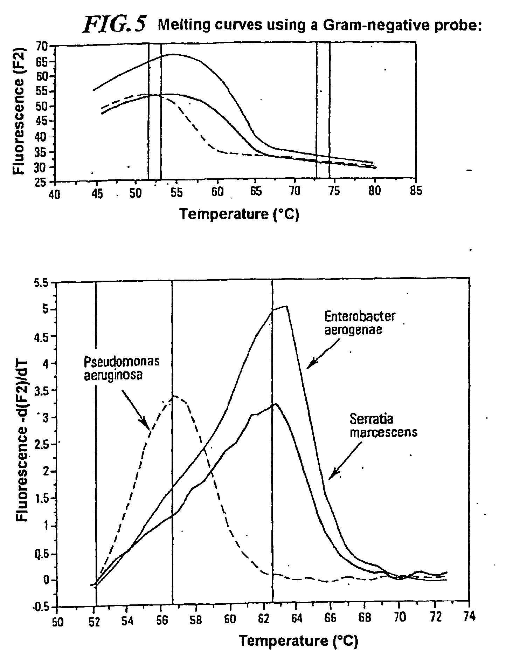

Fig. 5 stellt dar, daß sich bereits bei einem (n = 1) Mismatch (Pseudomonas aeruginosa) eine deutlich niedrigere Schmelztemperatur ergibt als bei einem kompletten Match der Sonde (Enterobacter aerogenae und Serratia marcescens).

Nachfolgend soll anhand von Ausführungsbeispielen die Erfindung näher erläutert werden.

### A. Qualitative Bestimmung von Mikroorganismen.

### Beispiel 1:

### Isolation der DNS aus Vollblut:

Zur Isolation der freien bakteriellen DNS aus Patientenplasma wird Vollblut in ein EDTA-Vollblutröhrchen (3,2 ml; Sarstedt) gesammelt. Das Vollblut wird sofort nach der Abnahme bei 2000g 3min zentrifugiert. Das überstehende Plasma wird von den zellulären Bestandteilen vorsichtig abgezogen. Das Plasma wird entweder direkt weiter bearbeitet, oder bei - 25°C .gelagert.

Die DNS-Präparation erfolgt einheitlich mit dem Nucleinsäure-Isolations-Kit "High Pure viral Nucleic Acid Kit" der Firma Roche Molecular Biochemicals. Die Zentrifugationsschritte werden in einer Zentrifuge der Firma Heraeus durchgeführt. Für die Inkubationen steht ein Thermomixer von Eppendorf zur Verfugung. Gearbeitet wird mit Pipetten von Eppendorf. Alle Pipettenspitzen sind autoklaviert und mit Filtern versehen. (Das Prinzip ist von Vogelstein, B. u. Gilles in D. Proc. Natl. Sci. USA 76, 615 - 619 beschrieben, worauf in vollem Umfang bezug genommen wird.).

Folgendes Standardprotokoll wird zur Gewinnung von DNS aus 200 ul Plasma angewandten einem 1,5 ml Eppendorf Reaktiosgefäß werden 250 ul Plasma mit 200 ul Bindepuffer (6M Guanidiniumchlorid, 10 mmol Harnstoff, 10mM Tris-HCl, 20% Triton X-100(v/v) in 25 ml, pH 4,4 (25°C)), und 40 ul Proteinase K-Lösung (90 mg lyophilisierte Proteinase K + 4,5 ml bidest. H2O) vermischt und 10 min bei 72øC inkubiert. Dann wird die so behandelte Probe mit 100 ul Isopropanol versetzt und gut gemischt. Die Probe wird in das obere Reservoir der High Pure Filter-Tube pipettiert und 1 min bei 8000 x g zentrifugiert. Der Waschschritt mit i-Propanol wird wiederholt. Die Elution der im Tube gebundenen Nukleinsäuren erfolgt mit 50 ul Elutionspuffer ebenfalls für 1 min bei 8000 x g. Die im Aufanggefäss erhaltene DNS-Lösung wird entweder bei - 25°C gelagert oder sofort für die PCR verwandt.

### Dekontamination des Mastermixes:

Die 16 S RNS ist innerhalb des bakteriellen Genoms eine hochkonservierte Region und eignet sich daher sehr gut für den allgemeinen Nachweis mikrobieller DNS durch eine Polymerase-Kettenreaktion (PCR). Kontaminierende bakterielle DNS von recombinant hergestellten Reagenzien wird allerdings ebenso mitamplifiziert und führt zu einem "Hintergrundrauschen" des Verfahrens aufgrund der Amplifikation kontaminierender mikrobieller DNS.

Aufgrund der rekombinanten Herstellung aller kommerziellen Taq-Polymerasen in einem bakteriellen Wirt und einer unzureichenden Aufreinigung des Enzyms, stellt die Taq-Polymerase selbst die Hauptkontaminationsquelle von mikrobieller genomischer DNS dar. Eine Dekontamination der Polymerase und des Mastermixes, welcher die wesentlichen Reagenzien der PCR enthält, vor Zugabe der Template DNS ist notwendig, um das sogenannte Hintergrundrauschen der Methodik zu verringern.

Alle folgenden Reagentien stammen, wenn nicht anders angegeben, aus dem SYBR Green 1 Kit (Roche). Ansatz des Mastermixes zur Dekontamination in einem sterilen 1,5 mL Eppendorf Reaktiosgefäß:
9,4 ul ddH20)
2,4 ul MgCl2 -Stammlösung)
2,0 ul CYBR-Green 1 Mastermix)
0,2 ul (entspr. 1 Einheit) Enzym Mbo 1 (New England Biolabs)

Der Ansatz wird kurz gevortext und zentrifugiert (13000g , 10 s). Es folgen 45 min Inkubation bei 37øC, in denen das Restriktionsenzym kontaminierendes bakterielles Template schneidet, das so in der folgenden PCR nicht mehr amplifiziert werden kann. Nach dieser Dekontamination wird die Restriktionsendonuklease bei 95øC 3 min inaktiviert - Abkühlen auf Eis. Danach werden je 0,5 ul (entsp. 10 pMol) PCR-Primer AR3 und AR4 - diese schließen keine hypervariable Region ein - hinzugefügt Diese hier verwendeten Primer werden von Biomol (Belgien) bezogen und sind HPLC gereinigt.

Die Primersequenzen lauten wie folgt:

| | |
|---|---|
| Vorwärts: | AR3: 5'-gcg gtg aaa tgc gta gag at-3' |
| Rückwärts: | AR4: 5'-gtt tac ggc gtg gac tac ca-3' |

Der Mastermix wird anschließend kurz gevortext und zentrifugiert (13000g, 10 s). Danach werden 15 ul des dekontaminierten Mastermixes in die LightCycler-Kapillare überführt und 5 ul Proben-DNS (oder Standard-Verdünnungs-Proben, Sau.) hinzugefügt. Die Kapillaren werden danach verschlossen, bei 1000g 10 sec zentrifugiert und in den LightCycler-Rotor eingesetzt

### Beispiel 2

### Aufbereitung einer Patientenprobe (BAL)

Es wurde bronchioalvaeoläre Lavage (BAL) mit Wasser - dies bedeutet, daß Wasser über ein Bronchoskop in die Lunge zum Spülen eingebracht und dann wieder abgesaugt wird, aufbereitet. Dazu wurden 200 µl BAL mit dem High Pure Viral Nucleic Acid Kit der Fa.ROCHE aufgearbeitet - d.h. sie werden mit 200µl Arbeitslösung, bestehend aus 200 µl Bindepuffer (6M Guanidiniumchlorid, 10 mmol Harnstoff, 10mM Tris-HCl, 20% Triton X-100(v/v) in 25 ml, pH 4,4 (25°C)), in dem 2 mg poly(A)carrier-RNA-Lyophilisat gelöst sind, versetzt und anschließend mit 40 µl Proteinase K-Lösung (90 mg lyophilisierte Proteinase K + 4,5 ml bidest. H2O). Nach Zugabe der wässrigen Proteinase-K-Lösung wird sofort gut gemischt und 10 min bei 72 °C inkubiert. Nach Inkubation werden die Proben mit 100 u1 Isopropanol p.A. versetzt und gut gemischt. Nun wird ein high Pure Filter-Tube - mit speziell aufbereitetem Glasmaterial - Polypropylengefäße mit einer Aufnahmekapazität von bis zu 700 ul Probevolumen, die Glasfaservlies enthalten - in ein 2 ml Polypropylen-Aufanggefäß eingesetzt und die Probe in das obere Reservoir pipettiert..Das Auffanggefäß mit Filter-Tube und Probe wird in einer Standard-Tischzentrifuge 1 min mit 8000 x g zentrifugiert. Der aus dem Filter-Tube Röhrchen austretende Durchlauf wird verworfen - die DNS bleibt in dem Filter-Tube Röhrchen gebunden. Das Filter-Tube wird in ein neues Auffanggefäß eingesetzt. Die daran gebundene DNS wird mit 450 ul Waschpuffer (20mM NaCl, 20mM Tris HCl, pH 7,5(25°C) in 40 ml abs.Ethanol p.A.+ 10 ml Wasser), der in das obere Reservoir pipettiert und durch Zentrifugation über 1 min bei 8000 x g durch das Röhrchen der Tube gedrückt wird, gewaschen. Dieser Waschschritt wird mit 450 ul Waschpuffer in einem neuen Auf anggefäß wiederholt, wobei nun nach Abschluß der Zentrifugation bei 8000g 10s bei max. Geschwindigkeit der Zentrifuge - ca 13000xg zentrifugiert wird.

Das Auffanggefäß wird danach verworfen und das gewaschene Filter-Tube in ein neues, nuclase-freies 1,5 ml Reaktiosgefäß eingesetzt. Zur Elution der Nucleinsäuren von der Glasoberfläche werden nun 50 ul Elutionspuffer (nuclease-freies bidest. H2O), der auf 70°C erwärmt wurde), in das Filter-Tube pipettiert und 1 min bei 8000 x g zentrifugiert. Die austretende Nucleinsäure-Lösung wird sofort in der PCR weiterverarbeitet (oder für spätere Analysen bei 2- 8 °C gelagert).

Die Isolation der Nucleinsäuren kann aber mit jedem anderen geeigneten Verfahren stattfinden, so bspw. mit dem MAGNA-Pure-Gerät der Fa ROCHE, das automatische Extraktion von Nucleinsäuren aus Lösungen ermöglicht und dadurch Fehler, die durch eine unautomatisierte Probenname/Verarbeitung auftreten könnten, vermeidet. Die Isolation von Pilz-DNS aus Vollblut ist bspw. in der WO 97/07238 beschrieben, auf die Offenbarung wird in vollem Umfang bezug genommen.

### Beispiel 3

### Amplifikation der isolierten bakteriellen Nucleinsäuren mit gelabelten Konsensus-Primern und gelabelten gram+ spezifischen sowie gram-spezifischen Sonden

Jeweils 2ul Probelösung mit Bacteroides fragilis, Pseudomonas aeruginosa, Escherichia Coli und Klebsiella pneumoniae werden mit 2ul Mastermix, 2ul Fast Start Mix f. hybProbes-Roche, 2,4 ul 25mM MgCl2 in H20, 10 pmol PLK1 Primer und 8 pMol des fluoreszenzmarkierten Rückwärts-Primers IFL versetzt. Der Primer IFL ist an der vorletzten Thymidin-Base mit Fluoreszein gelabelt, so daß die amplifizierte DNS stets ein Fluoreszein-Label enthält, das nahe der spezifischen Region der 16S-Region eingebaut ist. Der FastStart Mix der Fa. Roche für die PCR, wird bevorzugt verwendet. Es werden 3 pmol gram(+)Sonde ISP2, die am letzten Guanidin mit LC705 gelabelt ist und 3 pmol der gram(-)Sonde ISN2, die mit LC640 am Thymidin des 3'-Endes gelabelt ist, zugesetzt und auf 18 ul mit dest. hochreinem H2O verdünnt.

Mastermix und Probe werden vereinigt, in PCR-Kapillaren eines Light-Cyclers gefüllt und durch Zentrifugation in einer Laborzentrifuge bei 800 x g über 1 min in die Kapillare herunterzentrifugiert. Anschließend wird die Kapillare in den Light Cycler eingesetzt und 45 Zyklen zwischen 95 und 52 °C gefahren.

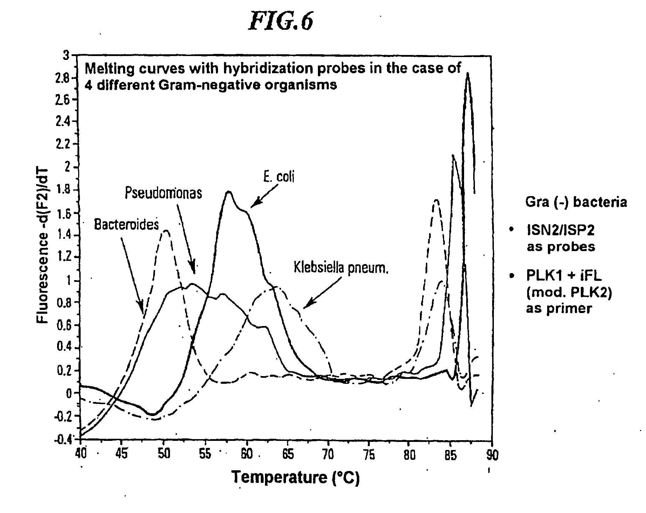

Im Anschluß an die Amplifizierung wird als Abschluß der PCR im Light Cycler eine PCR-Schmelzkurve unter Auswertung im 640 nm Kanal ermittelt. Die Schmelzkurven der verschiedenen Bakterien-DNS sind in Fig. 6 dargestellt. Die Fig.6 zeigt, daß die Messung in 640 nm Kanal die spezifisch durch die gram-Sonde erfaßten Amplifikationsprodukte ergibt, bzw. das Hybridisationsprodukt zwischen dieser Sonde und der Bakterien-RNS, wobei sich je nach unterschiedlichem gram (-) Keim eine unterschiedliche Schmelzkurve ergibt. Bakterien-DNS von gram(+)Bakterien kann im 705 nm Fenster bei Markierung der gram(+) Sonde in der gleichen Probe gemessen werden. Durch den Einsatz der verschieden gelabelten Sonden ist es möglich, mit 2 Sonden in einer Probe gram(+) und gram(-)Bakterien zu erkennen und aufgrund des Schmelzpunktes zu differenzieren.

Deutlich ist in Fig. 6 zu sehen, daß die Sonde mit unterschiedlichen Bakterien-RNS verschiedene Schmelzkurven liefert, die durch die Mismatches zwischen der Sonde und der DNS erklärbar sind. Durch die Unterschiede in den Schmelzkurvenformen mit gram(-) Bakterien-DNS läßt sich eine recht genaue Bestimmung des Bakteriums, das in der Lösung Probe anwesend war, durchführen. Hier tritt eine erste Schmelzkurve im Bereich von.40 - 55°C auf - d.h. das Abschmelzen der Sonde vom Bacteroides Amplifikat (Bacteroides) erfolgt hier früh, als nächstes folgt Pseudomonas mit einer zweiten, sehr breiten Schmelzkurve im Bereich von 45 - 65 °C. Aus der Kombination der Schmelzkurvenanalysen können hier die Keime verschiedener Bakterienarten weiter differenziert werden, ohne daß eine weitere PCR oder ein Öffnen der Kapillare zur Zugabe eines weiteren Reagens notwendig wäre.

### Beispiel 4

### Nachweis mehrerer Bakterienarten aus Reinzuchtstämmen nebenineinander,

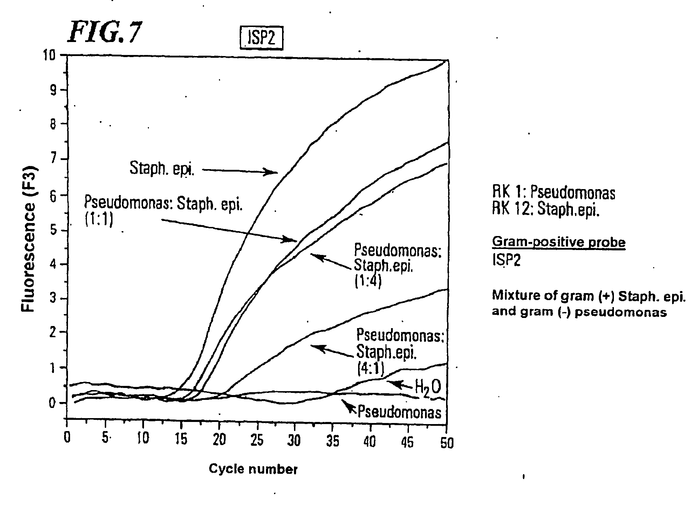

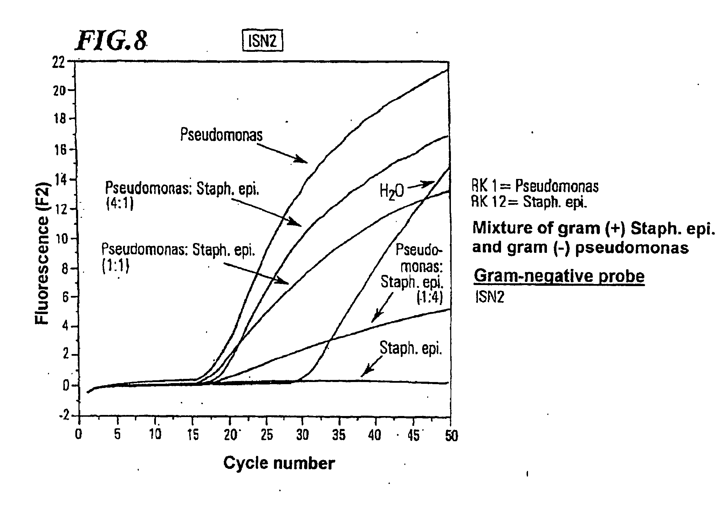

Es wurde eine Mischung 1:4 Pseudomonas mit Staph. epidermidis hergestellt und in Anwesenheit der Sonden ISP2 amplifiziert, wie im vorangehenden Beispiel angegeben. Die Schmelzkurve in Fig. 7 zeigt deutlich, daß ISP2 nur staph. epi. erkennen kann und daß das Signal von Staph. epi. durch pseudomonas nicht gestört wird. Das Vergleichbare ergibt sich aus Fig. 8, wo der gleiche Versuch mit ISN2 durchgeführt wurde- auch hier ergibt sich nur der gram(-) Mikroorganismus Pseudomonas, dessen Anzeige offensichtlich durch staph.epi. nicht gestört wird.

Der Nachweis verschiedener Spezies nebeneinander in einer Messung ist somit möglich.

### Beispiel 5

### Nachweis von Pilz-DNS aus Reinzuchtstämmen

Sonden/Primerkombinationen, die spezifisch für die Amplifikation/Detektion von Pilz-DNS eingesetzt werden können, befinden sich in der Liste 1. Die Methodik erfolgt analog zur Detektion der Bakterien. '

### Beispiel 6

### Nachweis und Bestimmung von Bakterien-rRNS aus Patientenproben

Es wurden jeweils 3 ml einer bronchioalvaeolären Lavage (BAL) mit Wasser - dies bedeutet, daß Wasser über ein Bronchoskop in die Lunge zum Spülen eingebracht und dann wieder abgesaugt wird, die von zwei Intensiv-Stations-Patienten P2 und P4, die an Lungenprobleme litten, stammte, 1:50 mit bidestilliertem Wasser verdünnt. Ergänzend ist festzustellen, daß bei Gesunden eine BAL keine Keime ergibt.

Die so verdünnte BAL-Flüssigkeit wurde mittels des High Pure Viral Nucleic Acid Kit, wie in Beispiel 2 angegeben, gereinigt.

Danach wurde die so aufbereiteten Probe in verschiedene Kapillaren aufgeteilt:

Jede Probe wurde im LightCycler mit Konsensus-Primern PLK1 und PLK2 in Gegenwart von SYBR Green amplifiziert und sodann nach 40 Zyklen im SYBR-Green-Format die Fluoreszenz der doppelsträngigen Amplifikate und deren Schmelzverhalten untersucht. Das Ergebnis ist in Fig. 1b dargestellt.

Wie durch Vergleich mit Fig. 1 ersichtlich, ist der Schmelzpunkt des amplifizierten doppelsträngigen DNS-Teils von P4, der mit SYBR-Green angefärbt wurde, gleich dem, der mit einer Reinzucht von Pseudomonas aeruginosa (ATCC 27852) gemessen wurde - er liegt im gleichen Bereich wie Enterococcus faecalis, Streptococcus pneum. und staph epidermidis - mittels der PCR/SYBR-Green Untersuchung konnten die möglichen Mikroorganismen auf wenige zu testende Keime eingeschränkt werden. Ein Vergleich mit den Ergebnissen der Mikrobiologie, die mehrere Tage nach der Probenahme diesen Keim nachweisen konnte, bestätigte die Ergebnisse der PCR/Schmelzpunktanalyse, die innerhalb einer Stunde vorlag.

Die Probe des Patienten P2 wurde ebenfalls mit den Konsensus-Primern PLK1 und PLK2 amplifiziert und sodann der Schmelzpunkt des gebildeten doppelsträngigen Produkts mit SYBR-Green untersucht - der Schmelzpunkt des amplifizerten Produktes entspricht demjenigen von Enterobacter cloacae und Serratia marcescens - wie aus Fig. 1a ersichtlich - die mikrobiologische. Untersuchung zeigte, daß enterobacter cloacae vorlag.

Demnach war es durch das erfindungsgemäße Verfahren möglich, innerhalb einer Stunde die tatsächlich spezifisch zu testenden möglichen Mikroorganismen im Hyb-Probe-Format stark einzuschränken, wobei dann eine genaue Bestimmung - bspw. mit spezifischen Sonden, innerhalb einer weiteren Stunde erfolgen kann und die Therapie schnell, auf den Mikroorganismus abgestellt, effektiv beginnen kann.

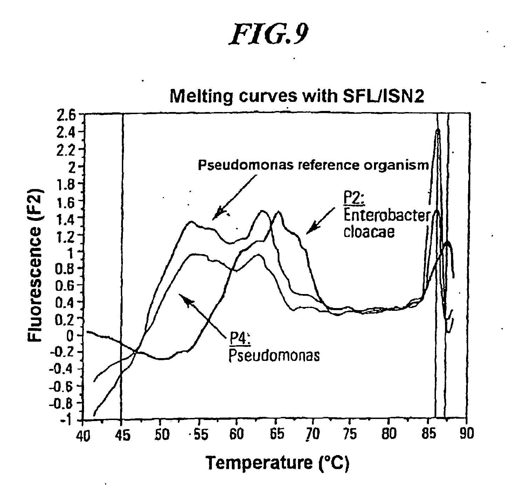

Die gleiche BAL-Proben von P4 und P2 wurden auch mit den Konsensus-Primern PLK1 und PLK2 sowie dem Sondenpaar ISN2/ISP2, versetzt und nach der Amplifikation der PCR im Light-Cycler, untersucht. Das Ergebnis der Schmelzpunktanalyse des Hybridisationsproduktes mit ISN2/ISP2 ist in Fig. 9 dargestellt. Deutlich ergibt sich, daß auch hier bei P4 nur eine Fluoreszenz der ISN2-Sonde (fluoresziert bei 640 nm) gemessen werden kann - wobei die Kurvenform der von Pseudomonas mit der ISN2-Sonde entspricht - wie sich aus dem Vergleich mit der Kurve mit dem Reinzuchtkeim in der gleichen Fig.9 sowie in Fig. 6 ergibt. Danach erschien die Anwesenheit von Pseudomonas wahrscheinlich. Das gleiche zeigt sich für die Schmelzpunkt. Somit ist es mittels der Sonde ISN2 möglich, nur gram-negative Bakterien aufzufinden - also die gram(+) Bakterien bereits auszuschließen - und dann aufgrund der speziellen Schmelzpunkte des Hybridisationsprodukts die Auswahl noch enger einzuschränken bzw. in vielen Fällen, den Mikroorganismus bereits in diesem Run direkt zu bestimmen.

Die Probe P2 zeigte eine Kurvenform, die der von Enterobacter cloacae ähnelte - tatsächlich konnte die Anwesenheit dieses Keims wieder durch die Mikrobiologie bestätigt werden.

Somit ist es erfindungsgemäß möglich, im SYBR-Green-Format über die Bestimmung der Schmelzpunkte des amplifizierten Produktes bereits eine Vorauswahl möglicher anwesender Keime zu treffen - im Sondenformat können mittels gruppenspezifischer Sonden engere Unterscheidungen möglicher Mikroorganismen innerhalb kürzester Zeit durchgeführt werden.

In einer weiteren PCR könnte dann - wie bspw. aus Light-Cycler-System - der Anleitung des Light-Cycler-Instrumentes - bekannt - der Mikroorganismus mittels spezifischer Sonden und deren Schmelzpunkt bestimmt werden. Durch das erfindungsgemäße Verfahren ist aber eine starke Einschränkung der tatsächlich mittels spezifischer Sonden durchzuführender Versuche möglich - bzw. bei vielen Keimen, deren Schmelzkurven so typisch verlaufen, daß keine weitere Schmelzkurve diesen Verlauf aufweist, kann eine weitere Nachbestimmung vermieden werden.

### B. Quantifizierung der DNS:

In vielen Fällen ist es erwünscht, feststellen zu können, ob die Keimbelastung der ursprünglichen Probe hoch war und somit auf eine akute Infektion hindeutet, oder aber ob sie eher gering ist - also eine abgeklungene Infektion oder aber eine beginnende Infektion andeutet. Es ist so auch festzustellen, ob es sich möglicherweise um eine übliche Besiedelung handeln könnte, oder ob diese Besiedelung mit Mikroorganismen pathogen ist. Dazu ist es notwendig, einen entsprechenden Vergleichsstandard herzustellen.

### Ansetzen einer Standard-Verdünnnungs-Reihe:

Für die quantitative Bestimmung bakterieller genomischer DNS ist es notwendig, eine Standard-Verdünnungs-Reihe mit bekannten DNS-Konzentrationen aus Template DNS herzustellen, die bei jeder PCR mitamplifiziert wird. Aus der Fluoreszenz der Standard-Verdünnungs-Proben ermittelt der LightCycler (Roche) eine Regressionsgerade, über welche die Konzentration der Proben berechnet wird.

Für die Standard-Verdünnungs-Reihe wurde genomische DNS von staphylococcus epidermidis photometrisch bei 260 nm quantifiziert. Hierbei entspricht eine Optische Dichte (OD) von 1,0 50 ul/ml doppelsträngiger DNS. Die Reinheit der Proben wird aus dem Verhältnis OD(260) : OD(280) ermittelt. Ein Verhältnis von 1,8 - 2,0 gilt als rein. Anschließend erfolgt eine Verdünnung auf 500, 100, 50, 10, 5 und 1 fg/ul. Von diesen wurde je 5 ul als Template dem PCR Mastermix zugesetzt.

### Herstellung eines Plasmid-Standards

Es hat sich herausgestellt, daß ein genomischer Standard nicht immer optimal zur Amplifikation ist, da aufgrund unterschiedlich methylierter Seitenketten keine vollständig einheitliche Amplifikation erfolgt. Daher wurde ein Plasmid-Standard erarbeitet.

Ein PLK-Produkt (180 Basenpaare) - d.h ein solches, das von den beiden PLK-Primern amplifiziert wird, wird in den Vektor pCR(r)4TOPO(r) der Fa. Invitrogen gemäß dem TOPO TA Cloning Kit for Sequencing einkloniert, in den Bakterien dieses Kits transformiert, dort amplifiziert und dann aus diesen Bakterien isoliert und quantifiziert (photometrisch bei OD 260 nm : 1 = 50ug/ml) - s. Maniatis oder aber Shuman, S.(1994), J. Bio. Chem. 269, 32678 - 32684 und Bernard, P. et. al. (1993) J. Mol. Bio.. 234,534-541)

Die Quantifizierung kann auch durch jegliches anderes geeignetes Verfahren, bspw. mit SYBR-Green nach dem Verfahren der FHG für Grenzflächenforschung, Stuttgart, Deutschland erfolgen. Das quantifizierte Produkt wird verdünnt und mit Wasser für die Eichkurve verwendet.

### Polymerase-Kettenreaktion (PCR):

Die PCR erfolgt auf einem LightCycler (Roche) unter Verwendung der Lightcycler Run Software 3.39. Es wurde zunächst eine initiale Denaturierung bei 95øC 30 sec mit 20°/sec Transition Rate durchgeführt. Es schlossen sich 45 Zyklen an mit:
Denaturierung 95°C, 1 sec, 20°/sec
Annealing 60°C, 5 sec, 20°/sec
Extension 72°C, 7 sec, 20°/sec
Measurement 83 °C, 0sec, 20°/sec, (Acquisition Mode: single) an.

Im Anschluß an die Amplifizierung wird das entstandene Produkt mittels Schmelzkurve analysiert. Die Bedingungen hierbei sind:
Denaturierung 95°C, 3 sec, 20°C/sec
Cooling 60°C, 30sec, 20°C/sec
Measurement 95°C, 0 sec, 0.1°C/sec, Acquisition Mode: continuous

Abschließend wurde die PCR durch einen Kühlschritt mit 40°C, 30sec, 20°/sec beendet.

### Datenanalyse / Quantifizierung

Für die Datenanalyse und Quantifizierung der bakteriellen DNS wird die halbautomatische Lightcycler Software 3.1.102 eingesetzt. Der Ablauf der Analyse und Quantifizierung erfolgt in drei Schritten und wird durch die Software vorgegeben:
1 Festlegung der Autofluoreszenz-Schwelle durch Software im Arrhythmetik-Modus
2. Festlegung des Noise-Bands manuell anhand der Kurven der Standard-Verdünnungs-Reihe. Das Noise-Band wird in den loglinearen Anteil der Standard-Verdünnungs-Reihe unmittelbar nach dem unteren Inflektionspunkt gelegt.
3. Die Quantifizierung der Proben erfolgt anhand einer Regressionsgeraden, die durch die LightCycler-Software anhand der Standard -Verdünnungs-Reihe ermittelt wird.

### Beispiel 7

### Bestimmung bakterieller DNS im Plasma von Sepsis-Patienten mit Blottechnik/PCR

Es wurden die gleichen Verfahrensschritte, wie bei Beispiel 2 am anhand von Plasma von Sepsis-Patienten durchgeführt, allerdings erfolgte die Dekontamination der PCR-Reagentien auf die nachfolgende Weise:

### Reinigungsverfahren

Da die für die PCR verwendeten Polymerasen meist bakteriellen. Ursprungs sind und daher häufig mit bakterieller DNS verunreinigt sind, ist es in der Regel notwendig, die Verunreinigung vor der unspezifischen PCR unschädlich zu machen, um zu verhindern, daß die verunreinigende DNS mitamplifiziert wird und so zu einem falsch-positiven Ergebnis führt. Es ist daher sinnvoll, hochreine Polymerase einzusetzen bzw., falls diese den Anforderungen nicht genügt, die in der PCR Amplifizierung eingesetzte Polymerase vor dem Einsatz zur Vervielfachung der mikrobiellen DNS/RNS zu dekontaminieren, bspw. durch Restriktionsenzym-Be-handlung, durch Methoxypsoralen/UV-Bestrahlung oder andere geeignete Verfahren

### Reinigung der bakteriellen PCR-Reagentien durch Methoxypsoralen-Behandlung

Die Quantifizierung mikrobieller DNS/RNS by PCR - Amplification ist sinnvoll, wobei aber die Verunreinigung der PCR-Reagentien mit mikrobieller DNS ein schwerwiegendes Problem darstellt, die zu falsch positiven Resultaten führen, wenn ein universeller 16S rRNS Primer eingesetzt wird. Hier wird 8-Methoxypsoralen kombiniert mit UV-Bestrahlung zur Inaktivierung verunreinigender DNS eingesetzt.

Es wird die Wirkung von 8-Methoxypsoralen auf die PCR-Agentien, insbesondere auf das Hintergrundrauschen verunreinigender 16S rRNS unter Verwendung eines Echtzeit-PCR-Systems zur Detektion mikrobieller DNA im Plasma von Sepsis-Patienten untersucht. Auch hier wurden wieder alle PCR-Reaktionen auf dem Lightcycler Instrument (Roche) durchgeführt. Quantifizierung wurde gemäß den SYBR Green I-Verfahren durchgeführt. Zur Dekontamination wurden 25 ug/ml 8-Meth-oxypsoralen (Sigma) and 5 min UV-Bestrahlung (366nm) eingesetzt. Das 8-Meth-oxypsoralen wurde in Dimethylsulfoxid 2,5 %, gelöst. Durch die Dekontamination der PCR-Agentien kann nun 16S rRNS von verstärkter Staphylococcus epidermidis DNS durch die Schmelzkurvenanalyse unterschieden werden (Schmelzpunkt der verunreinigenden E.Coli-DNS 89°C; Schmelzpunkt der Staphylococcus epidermidis DNS: 87°C. Die Behandlung der PCR-Regentien mit 8-Methoxypsoralen und UV-Bestrahlung führte zur Elimination von bis zu 800 Kopien bakterieller DNS, wobei 400 Kopien, die zu einer Probe nach dem Dekontaminationsverfahren zugesetzt wurden, ohne Koamplifikation der verunreinigenden DNS amplifizert werden konnten.

Die Behandlung der PCR-Reagentien mit 8- Methoxypsoralen und UV führt demzufolge zu einer zufriedenstellenden Elimination von verunreinigender mikrobieller DNS. Die übrigen Schritte wurden wie bei Beispiel 1 durchgeführt.

### Beispiel 8

Die Verfahren wurden, wie in Beispiel 1 durchgeführt, allerdings erfolgte die Dekontamination der PCR-Agentien wie folgt:

### Reinigung der PCR-Agentien durch DNAse

Ein alternatives Reinigungsverfahren für die PCR-Reagentien liegt im Einsatz von DNAse. Zur Reinigung der PCR-Agentien, die zu falsch positiven Ergebnissen bei einer Real-Time PCR führen und aus mikrobieller DNS bei der Herstellung der PCR-Reagentien stammen, werden die PCR-Reagentien 15 min mit DNAse behandelt. Danach wurde die DNAse durch Wärmebehandlung bei 95øC über 50 min inaktiviert. Deep-Vent-Exo(-) polymerase and Ampli-Taq Polymerase wurden für die Amplifikation mit Breitband-Primern von konservierter mikrobieller 16S ribosomaler DNS Nucleotid Sequenzen durch Polymerase Chain Reaction verwendet. Die derart dekontaminierten PCR-Agentien ermöglichten einen Ausschluß der verunreinigenden bakteriellen DNS aus den PCR-Reagentien.

Es konnten mit den erfindungsgemäß gereinigten PCR-Reagentien Bakterien-DNS-Konzentrationen von nur 6 pg/ml bestimmt werden.

### Beispiel 9:

### Grobe Identifikation bakterieller DNS durch DNS-Schmelzkurven verschiedener Bakterien (SYBR-Green)

Die gram-negativen Bakterien zeigten eine Schmelztemperatur zwischen 89.7 und 89.1°C, während die gram-positiven Bakterien eine Schmelztemperatur zwischen. 87,9 und 87, 6°C aufwiesen. E.coli zeigt die gleiche Schmelztemperatur wie die die Taq-Polymerase verunreinigende DNS. Dieses weist darauf hin, daß die verunreinigende DNS aus von E-coli stammenden Polymerasen stammt. Im vorliegenden Fall zeigten Amplifikationsprodukte von Patienten mit Sepsis Schmelzkurven, die sich von diesem Hintergrundsignal unterschieden. Diese Kurven zeigten Schmelztemperaturen von 88.1 und 87.2 °C. Die Identifizierung der Bakterien kann durch Hybridisierung Fluoreszenzfarbstoff-markierter Oligonucleotide mit der amplifizierten bakteriellen DNS verfeinert werden.

Demzufolge ist die DNS-Schmelzpunktanalyse in Kombination mit der Hybridisierung spezifischer, Fluoreszenzfarbstoff-gekoppelter Oligonucleotide mit dem PCR-Produkt ein ausreichendes Verfahren, um Bakterienspezies sehr schnell zu unterscheiden und zu bestimmen.

Bestimmung von Bakterien durch Kombination von PCR in Gegenwart von verschiedenen mehrfachspezifischen Hybridisierungssonden

### Nachweis von E.Coli

Es wurde eine PCR mit genomischer DNA von E. Coli mit dem Primer PLK1 und dem mit Fluoreszein markierten Primer IFL in Gegenwart der mit RRC markierten Sonden ISN2 im LightCycler der Fa Roche über 45 Zyklen durchgeführt. Nach Abschluß der Zyklen wurde eine Schmelzkurve der hybridisierten Produkte amplifizierte bakterielle DNS durchgeführt. Es zeigte sich eine Schmelzkurve des Hybridisationsprodukts mit der Sonde für gram-Bakterien bei der Wellenlänge 705nm, was anzeigte, daß DNS eines gram-negativen Bakteriums vorhanden war. Das Maximum der df/dt-Kurve - d.h. der Schmelzpunktbereich des Hybridisationsproduktes E.coli mit der Sonde ISN2 lag hier zwischen etwa 52 und 66°C.

Die Betrachtung der gleichen Schmelzkurve bei 640 ergab keine Schmelzkurve - die gram(+) Sonde hatte also keine DNS grampositiver Bakterien gefunden und lieferte daher kein Signal. (s. Fig.6)

### Nachweis von Staph. epidermidis

Der gleiche Versuch wie unter 1 wurde mit staph.epi DNS durchgeführt. Es konnte ein Schmelzbereich des Hybridisationsprodukts mit der gram(+) Sonde ISP2 von zwischen 50 - 64°C gefunden werden (s. Fig. 7). Die gram(-) Sonde ISN2 ergab, da keine DNS gram-negativer Bakterien vorhanden war, kein Signal.

Selbstverständlich ist es immer möglich, nach einer derartigen Vorklassifizierung die Auswahl nochmals mittels Hybridisierung mit einer spezies-spezifischen Sonde nachzuprüfen, um die Sicherheit des Verfahrens zu erhöhen.

### Spezifizierung der amplifizierten PCR-Produkte

Ein Beispiel für die im zweiten Schritt der Methodik durchgeführten Identifikation der Keimspezies ist die Technik des reversen Dot Blots. Die Spezifizierung der Keime ist nicht auf diese Technik beschränkt sondern auch zum Beispiel mittels DNS-Mikrochip-Technik durchführbar.

### Beispiel 10:

### Spezifizierung der amplifizierten DNS/RNS mit Reversem Dot Blot:

Es wurden verschiedene Mikroorganismen, Candida albicans;Candida krusei ; Candida parapsilosis ; Candida tropiealis; Torulopsis glabrata; Aspergillus; E. coli Staph. epi ; Pseudomonas; Acinobacter baumanii; Staph. Aureus; Enterococcus faecium; Enterococcus faecialis; Haemophilus influ.;Enterobacter cloacae; Klebsiella pneumoniae; Serratia;Legionella pneu. Proteus vulgaris; Bacteroides fragilis; Streptococcus pyogenes; Mycobact. pneum.; Corynebact. Jejuni; Enterobacter aerog.Mycobact. tuberculosis wie aus der Liste 1 ersichtlich, nach Amplifizierung ihrer DNS/RNS über spezifische Sonden bestimmt.

Das PCR-Produkt der Quantifizierungsreaktion von bakterieller DNA wird während der PCR-Reaktion durch Einbau von Digoxigenin-markiertem dUTP markiert (die Amplifikation von Pilz-DNA wird anhand der in Liste 1 angegebenen Konsensus-primer PFUI und PFU2 durchgeführt). Das PCR-Produkt dient nach seiner Quantifizierung durch Real Time PCR zur Hybridisierung mit auf einer positiv geladenen Nylonmembran fixierten keimspezifischen Oligonucleotidsonde (reverser Dot Blot). Die positive Bindung des PCR-Produktes an eine spezifische Sonde (Sondendesign für die Bakterienspezies und Pilzart: s. Liste 1) wird anhand einer Antikörperbindung und Substratreaktion durch Chemilumineszenzsignal auf einem Röntgenfilm detektierbar.

Benötigte Materialien:
- Falkon-Röhrchen (50/25ml);
- Nylonmembran (bspw. Roche Diagnostics) (ca. 5 x 8 cm2).

Die entsprechenden Oligonucleotid-Sonden in einer Konzentration von 100 pMol/ul und das PCR-Produkt, durch Dig-dUTP-Einbau markiert (Positivkontrolle für die Detektion) wurden je 2ul auf die trockene Nylonmembran aufgebracht. Die Nucleinsäuren wurden auf der Nylonmembran durch UV-Bestrahlung (Crosslinking mit UV-Licht (302 nm, 400 mJoule/cm2) fixiert.

### Prähybridisierung: (1 Stunde)

30 ml Standardhybridisierungspuffer wurden in Falkon-Röhrchen gefüllt, die Membran eingelegt, und 1 Std. bei Hybridisierungstemperatur (50°C) inkubiert.

### Hybridisierung: (1 Stunde)

Die DNS des zu spezifizierenden Dig-dUTP-markierten PCR-Produktes wurde 10 min bei 95°C hitzedenaturiert, dann auf Eis gekühlt. Falls die eingefrorene (-20øC) Hybridisierungslösung mit markierter DNA wiederverwendet werden soll, wird diese ebenfalls vorher 10 min bei 95øC denaturiert.

Der Prähybridisierungspuffer wird verworfen. Das Filter darf bis zur Hybridisierung nicht trocken werden. 6 ml erwärmter Standardhybridisierungspuffer + 40 ul PCR-Produkt werden in Falkon-Röhrchen gefüllt und 1 h inkubiert. Danach wird 2 x 5 min bei Raumtemperatur mit 2 x Waschlösung (2 x SSC, 0,1% SDS) gewaschen, sodann 2 x 15 min bei Hybridisirungstemperatur mit 0.5 x Waschlösung (0.5 x SSC, 0.1% SDS). Danach wird die Nylonmembran luftgetrocknet oder weiter detektiert.

Für die Hybridisierung verwendete Puffer:
Hybridisierungspuffer: 5 x SSC, 0,1% N-Lauroylsarkosin, 0,021/o SDS, 1% Blockierungsreagenz (Kasein in Maleinsäurepuffer), bei 70°C gelöst
Maleinsäurepuffer: 0,1 M Maleinsäure, 0,15 NaCl, auf pH 7,5 mit NaOH eingestellt.

### Detektion des spezifisch an die Nylonmembran gebundenen PCR-Produktes

Alle Inkubationen erfolgen bei Raumtemperatur, wenn nicht anders angegeben. Die Membran wird kurz (1 - 3 min) in Waschpuffer gewaschen, 30 min im Blockierungspuffer leicht schütteln) inkubiert; danach 30 min in 30 ml Blockierungspuffer unter Zugabe von 3ul Anti-DIG-AP (Verdünnung-1:10.000) inkubiert. Dann wird die Membran zwei mal jeweils 15 min in Waschpuffer gewaschen, danach 2 min im Detektionspuffer. Dann wird die Membran (bis auf eine Seite !) eingeschweißt.

In einem sterilen Eppendorfröhrchen werden 990 ul Detektionspuffer + 10 ul CSPD (Box im Gefrierschrank) gemischt und mit der sterilen Pipette gleichmäßig auf die Membran aufgebracht.

Die zugeschweißte Membran wurde 15 min bei 37°C inkubiert. Dann wurde die eingeschweißte Membran in eine Belichtungskassette eingelegt, ein Röntgenfilm auf gelegt (im Dunkeln), markiert und verriegelt. Die Belichtung des Röntgenfilms wird 20-30 min durchgeführt. Nach der Belichtungszeit wird der Röntgenfilm entwickelt. Der Keimbefund wird anhand des Chemilumineszenz-Signals als dunkler Punkt (Dot) auf dem Röntgenfilm abgelesen.

### Zur Detektion verwendete Puffer

| | |
|---|---|
| Waschlösung | 0;3% Tween 20 in Maleinsäure-Puffer |
| Maleinsäurepuffer: | 0,1M Maleinsäure, 0,15M NaCl, pH 7,5 mit NaOH einstellen |
| Blockierungspuffer: | 10% Blockierungsreagenz in Maleinsäurepuffer |
| Detektionspuffer: | 100 mM TRIS-HCl, pH 9.5; 100 mM NaCl |

Während die Erfindung anhand bevorzugter Ausführungsbeispiele erläutert wurde, ist sie keineswegs auf diese beschränkt, sondern erstreckt sich auf alle dem Fachmann geläufigen, unter den Schutzbereich der Ansprüche fallenden Ausführungsformen.

## Patentansprüche

1. Verfahren zur Bestimmung mikrobieller DNS/RNS, **gekennzeichnet durch**:
- Vorlegen einer Probe mit RNS/DNS;
- Anreichern der DNS/RNS der Probe mittels eines PCR-Verfahrens mit generischen Konsensus-Primern
- Zugabe von mindestens einem markierten Oligonukleotid und temperaturabhängige Hybridisierung
- Bestimmung der DNS/RNS aufgrund der physikalischen Eigenschaften des DNS/RNS/Oligonukleotidkomplexes, wie der Temperaturabhängigkeit der Hybridisierung,
**dadurch gekennzeichnet, dass**
mindestens ein markiertes Oligonukleotid ausgewählt wird aus einer Gruppe von Oligonukleotiden, wobei diese Gruppe besteht aus:
ISN2 (= ACCGCAGAATAAGCACCGGCTAACTCCGTGCX), X = LC 640
ISN2* (= CCGCAGAATAAGCACCGGCTAACTCCGTX), X = LC 640
ISN (= AGAAGCACCGGCTAACTCCGXTGC), X = LC 640
ISP2* (= CCTAACCAGAAAGCCACGGCTAACTACGTGX), X = LC 705
ISP2 (= CCTAATCAGAAAGCGACGGCTAACTACGTGCX), X = LC 705
ISP (= AGAAAGCCACGGCTAACTACGXTGC), X = LC 705

2. Verfahren nach Anspruch 1, **dadurch gekennzeichnet, dass** mindestens eine weitere Sonde oder ein Primer ausgewählt werden aus einer Gruppe von Oligonukleotiden, wobei dieses Gruppe besteht aus:
PLK2H (= TACGGGAGGCAGCAGT)
REViFL (= GTATTACCGCGGCTGCXTG), X = FLUORESCEIN
Primer iFL (= TATTACCGCGGCTGCXTG), X = FLUORESCEIN

3. Verfahren nach Anspruch 1-2, **dadurch gekennzeichnet, dass** das PCR-Verfahren ein real-Time PCR-Verfahren ist und damit die Menge mikrobieller DNS/RNS in der Ausgangsprobe ermittelt wird.

4. Verfahren nach Anspruch 1-3, **dadurch gekennzeichnet, dass** zur Bestimmung der mikrobiellen DNS/RNS über die Schmelzkurve eine Vorklassifizierung des mindestens einen Mikroorganismus durchgeführt wird.

5. Verfahren nach einem der vorangehenden Ansprüche, **dadurch gekennzeichnet, dass** die in der PCR verwendeten Polymerasen vor dem Einsatz zur Vervielfachung der mikrobiellen DNS/RNS dekontaminiert werden, wie durch Restriktionsenzym-Behandlung, durch Methoxypsoralen/UV-Bestrahlung.

6. Verfahren nach einem der vorangehenden Ansprüche, **dadurch gekennzeichnet, dass** die zu untersuchende Probe Körperflüssigkeit, Abstriche oder Gewebe ist.

7. Kit zur schnellen Bestimmung von mikrobieller DNS/RNS in Proben, **gekennzeichnet durch**: dekontaminierte Polymerase; und Lösung mit einer Standard-DNS/RNS-Konzentration einer bekannten (Template) DNS/RNS,
**dadurch gekennzeichnet, dass** er ferner mindestens ein ggf. gelabeltes Konsensus-Primer-Paar, insbesondere mit einer Länge von etwa bis 23 Basenpaare aufweist.
- **dadurch gekennzeichnet, dass** er ferner RNS oder DNS-Sonden aufweist, die gelabelt sein können
- **dadurch gekennzeichnet, dass** mindestens eine Sonde ausgewählt werden aus einer Gruppe von Oligonukleotiden, wobei diese Gruppe besteht aus:
ISN2
ISN2*
ISN
ISP2*
ISP2
ISP

8. Kit nach Anspruch 8, **dadurch gekennzeichnet dass**, mindestens eine weitere Sonde oder ein Primer ausgewählt werden aus einer Gruppe von Oligonukleotiden, wobei diese Gruppe besteht aus:
PLK2H
REViFL
Primer IFL

## Claims

1. Method for determining microbial DNA/RNA, **characterized by**:
- providing a sample containing RNA/DNA
- enriching the DNA/RNA in the sample by means of a PCR process using generic consensus primers
- addition of at least one labelled oligonucleotide and temperature-dependent hybridization
- determining the DNA/RNA on the basis of the physical properties of the DNA/RNA/oligonucleotide complex such as the temperature-dependence of the hybridization **characterized in that**
at least one labelled oligonucleotide is selected from a group of oligonucleotides wherein this group comprises:
ISN2 (=ACCGCAGAATAAGCACCGGCTAACTCCGTGCX), X = LC 640
ISN2* (=CCGCAGAATAAGCACCGGCTAACTCCGTX), X = LC 640
ISN (=AGAAGCACCGGCTAACTCCGXTGC), X = LC 640
ISP2* (=CCTAACCAGAAAGCCACGGCTAACTACGTGX), X = LC 705
ISP2 (=CCTAATCAGAAAGCGACGGCTAACTACGTGCX), X = LC 705
ISP (=AGAAAGCCACGGCTAACTACGXTGC), X = LC 705

2. Method according to claim 1, **characterized in that** at least one additional probe or a primer is selected from a group of oligonucleotides wherein this group comprises:
PLK2H (=TACGGGAGGCAGCAGT)
REViFL (=GTATTACCGCGGCTGCXTG), X = fluorescein
Primer iFL (=TATTACCGCGGCTGCXTG), X = fluorescein

3. Method according to claims 1-2, **characterized in that** the PCR method is a real time PCR method and is used to determine the amount of microbial DNA/RNA in the initial sample.

4. Method according to claims 1-3, **characterized in that** a preclassification of the at least one microorganism is carried out in order to determine the microbial DNA/RNA by means of the melting curve.

5. Method according to one of the previous claims, **characterized in that** the polymerases used in the PCR are decontaminated for example by restriction enzyme treatment, by methoxypsoralen/UV radiation before being used to amplify the microbial DNA/RNA.

6. Method according to one of the previous claims, **characterized in that** the sample to be examined is body fluid, smears or tissue.

7. Kit for the rapid determination of microbial DNA/RNA in samples, **characterized by**: decontaminated polymerase, and a solution containing a standard DNA/RNA concentration of a known (template) DNA/RNA,
**characterized in that** it additionally has at least one optionally labelled consensus primer pair especially having a length of about 23 base pairs,
- **characterized in that** it additionally has RNA or DNA probes which can be labelled
- **characterized in that** at least one probe is selected from a group of oligonucleotides wherein this group comprises:
ISN2
ISN2*
ISN
ISP2*
ISP2
ISP

8. Kit according to claim 7, **characterized in that** at least one additional probe or a primer is selected from a group of oligonucleotides wherein this group comprises:
PLK2H
REViFL
primer iFL.

## Revendications

1. Procédé de détermination d'ADN/ARN microbien, **caractérisé par**
- la présence d'un échantillon qui contient de l'ADN/ARN,
- l'enrichissement de la teneur en ADN/ARN de l'échantillon au moyen d'une méthode PCR, avec des amorces consensus génériques,
- l'addition d'au moins un oligonucléotide marqué et hybridation dépendante de la température,
- la détermination de l'ADN/ARN à partir des propriétés physiques du complexe ADN/ARN/oligonucléotide, comme la variation de l'hybridation en fonction de la température,
**caractérisé en ce que**
au moins un oligonucléotide marqué est choisi dans le groupe d'oligonucléotides constitué par :
ISN2 (=ACCGCAGAATAAGCACCGGCTAACTCCGTGCX), X = LC 640
ISN2* (= CCGCAGAATAAGCACCGGCTAACTCCGTX), X = LC 640
ISN (= AGAAGCACCGGCTAACTCTCCGXTGC), X = LC 640
ISP2* (= CCTAACCAGAAAGCCACGGCTAACTACGTGX) X = LC 705
ISP2 (= CCTAATCAGAAAGCGACGGCTAACTACGTGCX), X = LC 705
ISP (= AGAAAGCCACGGCTAACTACGXTGC), X = LC 705

2. Procédé selon la revendication 1, **caractérisé en ce qu'**au moins une autre sonde ou une amorce est choisie dans le groupe d'oligonucléotides constitué par :
PLK2H (= TACGGGAGGCAGCAGT)
REViFL (= GTATTACCGCGGCTGCXTG), X = FLUORESCEINE
Amorce iFL (= TATTACCGCGGCTGCXTG), X = FLUORESCEINE

3. Procédé selon les revendications 1 à 2, **caractérisé en ce que** la méthode PCR est une méthode PCR en temps réel et que, la quantité d'ADN/ARN microbien est déterminée dans l'échantillon initial par celle-ci.

4. Procédé selon l'une quelconque des revendications 1 à 3, **caractérisé en ce que** pour la détermination de l'ADN/ARN microbien à l'aide de la courbe des points de fusion, on effectue une classification préalable du au moins un microorganisme.

5. Procédé selon l'une quelconque des revendications précédentes, **caractérisé en ce que** les polymérases utilisées dans la méthode PCR sont décontaminées avant leur mise en oeuvre dans l'amplification de l'ADN/ARN microbien, par exemple par traitement avec une enzyme de restriction, à l'aide de méthoxypsoralène/irradiation UV.

6. Procédé selon l'une quelconque des revendications précédentes, **caractérisé en ce que** l'échantillon à analyser est un liquide corporel, un frottis ou un tissu.

7. Trousse destinée à la détermination rapide d'ADN/ARN microbien dans un échantillon, contenant des polymérases décontaminées ; et une solution d'un ADN/ARN (matrice) connu en une concentration d'ADN/ARN étalon,
**caractérisée en ce qu'**elle contient en outre au moins une paire d'amorces consensus éventuellement marquées, en particulier ayant une longueur allant jusqu'à environ 23 paires de base,
- **caractérisée en ce qu'**elle contient en outre des sondes ADN ou ARN qui peuvent être marquées,
- **caractérisée en ce qu'**au moins une sonde est choisie dans un groupe d'oligonucléotides constitué par :
ISN2
ISN2*
ISN
ISP2*
ISP2
ISP.

8. Trousse selon la revendication 8, **caractérisée en ce qu'**au moins une sonde ou une amorce est choisie dans un groupe d'oligonucléotides, ce groupe étant constitué par :
PLK2H
REViFL
Amorce IFL.
